# EUROPEAN PATENT SPECIFICATION

(11) **EP 1 812 417 B1**
(45) Date of publication and mention of the grant of the patent: **29.10.2008**
(21) Application number: 05824228.0
(22) Date of filing: 17.11.2005
(51) Int. Cl.: C07D 311/60, C07D 311/78, C07D 311/80, C07D 493/04, A61K 31/352, A61K 31/35, A61P 5/28

(54) **NOVEL 2H-CHROMENE DERIVATIVES AS SELECTIVE ESTROGEN RECEPTOR MODULATORS**
NEUE 2H-CHROMENDERIVATE ALS SELEKTIVE ÖSTROGENREZEPTOR-MODULATOREN
NOUVEAUX DÉRIVÉS DE 2H-CHROMÈNE COMME MODULATEURS SÉLECTIFS DE RÉCEPTEURS D'ESTROGÈNE

(30) Priority: 18.11.2004 US 628987 P
(43) Date of publication of application: 01.08.2007
(73) Proprietor: Janssen Pharmaceutica NV, 2340 Beerse (BE)
(72) Inventor: JAIN, Nareshkumar F., Raritan, New Jersey 08869 (US); XU, Jiayi, Stanford, California 94305 (US); SUI, Zhihua, Raritan, New Jersey 08869 (US)
(74) Representative: Kirsch, Susan Edith
(86) International application number: PCT/US2005/041648
(87) International publication number: WO 2006/055694

(56) References cited:
- EP-A- 1 167 364
- WO-A-01/68634
- WO-A-03/044006
- WO-A-20/04050660

## Description

### FIELD OF THE INVENTION

The present invention is directed to novel 2H-chromene derivatives, pharmaceutical compositions containing them and their use to prepare medicaments useful in the treatment or prevention of disorders and diseases mediated by an estrogen receptor such as hot flashes, vaginal dryness, osteopenia, osteoporosis, hyperlipidemia, loss of cognitive function, degenerative brain diseases, cardiovascular, cerebrovascular diseases, hormone sensitive cancers and hyperplasia (in tissues including breast, endometrium, and cervix in women and prostate in men), endometriosis, uterine fibroids, osteoarthritis; and as contraceptive agents either alone or in combination with a progestogen or progestogen antagonist. The compounds of the invention are selective estrogen receptor modulators.

### BACKGROUND OF THE INVENTION

Estrogens are a group of female hormones essential for the reproductive process and for the development of the uterus, breasts, and other physical changes associated with puberty. Estrogens have an effect on various tissues throughout a woman's body, not only those involved in the reproductive process, such as the uterus, breasts, and external genitalia, but also tissues in the central nervous system, bones, the liver, skin, and the urinary tract. The ovaries produce most of the estrogens in women's body.

Menopause is defined as the permanent cessation of menses due to loss of ovarian follicular function and the almost termination of estrogen production. The midlife transition of menopause is characterized by a decrease in estrogen that provokes both short-term and long-term symptoms with the vasomotor, urogenital, cardiovascular, skeletal and central nervous systems, such as hot flushes, urogenital atrophy, increased risk of cardiovascular disease, osteoporosis, cognitive and psychological impairment, including an increased risk of cognitive disorders and Alzheimer's disease (AD).

Seventy-five percent of all women experience some occurrence of vasomotor symptoms associated with the onset of menopause such as body sweating and hot flushes. These complaints may begin several years before menopause and in some women may continue for more than 10 years either relatively constant, or as instant attacks without a definable, provoking cause.

Urogenital symptoms associated with the onset of menopause involving the vagina include a sensation of dryness, burning, itching, pain during intercourse, superficial bleeding and discharge, along with atrophy, stenosis. Symptoms involving the urinary tract include a burning sensation during urination, frequent urgency, recurrent urinary tract infections, and urinary incontinence. These symptoms have been reported to occur in up to 50% of all women near the time of menopause and are more frequent a few years after menopause. If left un-treated, the problems can become permanent.

Heart attack and stroke are major causes of morbidity and mortality among senior women. Female morbidity from these diseases increases rapidly after menopause. Women who undergo premature menopause are at greater coronary risk than menstruating women of similar age. The presence of serum estrogen has a positive effect on serum lipids. The hormone promotes vasodilation of blood vessels, and enhances the formation of new blood vessels. Thus the decrease in serum estrogen levels in postmenopausal women results in adverse cardiovascular effect. Additionally, it is theorized that differences in the ability of blood to coagulate may account for the observed difference in the occurrence of heart disease before and after menopause.

The skeleton is under a continuous process of bone degeneration and regeneration in a carefully regulated interaction among the bone cells. These cells are directly affected by estrogen. Estrogen deficiency results in a loss of bone structure, and decrease of bone strength. Rapid loss of bone mass during the year immediately following menopause leads postmenopausal osteoporosis and increased risk of fracture.

Estrogen deficiency is also one of the causes for the degenerative changes in the central nervous system and may lead to Alzheimer's disease and decline of cognition. Recent evidence suggests an association between estrogen, menopause, and cognition. More particularly, it has been reported that estrogen replacement therapy and the use of estrogen in women may prevent the development of AD, and improve cognitive function.

Hormone replacement therapy (HRT) - more specifically estrogen replacement therapy (ERT) - is commonly prescribed to address the medical problems associated with menopause, and also to help hinder osteoporosis and primary cardiovascular complications (such as coronary artery disease) in both a preventive and therapeutic manner. As such, HRT is considered a medical therapy for prolonging the average life span of postmenopausal women and providing a better quality of life.

ERT effectively relieves the climacteric symptoms and urogenital symptoms and has shown significant benefits in the prevention and treatment of heart disease in postmenopausal women. Clinical reports have shown that ERT lowered heart attack rates and mortality rates in populations that received ERT versus similar populations not on ERT. ERT initiated soon after menopause may also help maintain bone mass for several years. Controlled investigations have shown that treatment with ERT has a positive effect even in older women up to age of 75 years.

However, there are numerous undesirable effects associated with ERT that reduce patient compliance. Venous thromboembolism, gallbladder disease, resumption of menses, mastodynia, and a possible increased risk of developing uterine and/or breast cancer are the risks associated with ERT. Up to 30% of women who were prescribed with ERT do not fill the prescription, and the discontinuation rate is between 38% and 70%, with safety concerns, and adverse effects (bloating and break-through bleeding) the most important reasons for discontinuation.

A new class of pharmacological agents known as Selective Estrogen Receptor Modulators or SERMs have been designed and developed as alternatives for HRT. Raloxifene, a nonsteroidal benzothiophere SERM is marketed in the US and Europe for the prevention and treatment of osteoporosis under the trademark of Evista®. Raloxifene has been shown to reduce bone loss and prevent fracture without adversely stimulating endometrial and mammary tissue, though raloxifene is somewhat less efficacious than ERT for protecting against bone loss. Raloxifene is unique and differs significantly from ERT in that it does not stimulate the endometrium and has the potential for preventing breast cancer. Raloxifene has also demonstrated beneficial estrogen agonist effects on cardiovascular risk factors, more specifically through a rapid and sustained decrease in total and low-density lipoprotein cholesterol levels in patients treated with raloxifene. In addition, raloxifene has been shown to reduce plasma concentration of homocysteine, an independent risk factor for atherosclerosis and thromboembolic disease.

However, raloxifene has been reported to exacerbate symptoms associated with menopause such as hot flushes and vaginal dryness, and does not improve cognitive function in senior patients. Patients taking raloxifene have reported higher rates of hot flashes compared with either placebo or ERT users and more leg cramps than placebo users, although women who took ERT had a higher incidence of vaginal bleeding and breast discomfort than raloxifene or placebo users.

As yet, neither raloxifene nor any of the other currently available SERM compounds has been shown to have the ability to provide all the benefits of currently available ERT such as controlling postmenopausal syndrome and preventing AD, without causing adverse side effects such as increasing risk of endometrial and breast cancer and bleeding. Thus there exists a need for compounds which are selective estrogen receptor modulators and which provide all of the benefits of ERT while also addressing the vasomotor, urogenital and cognitive disorders or conditions associated with the decrease in systemic estrogen associated with menopause.

### SUMMARY OF THE INVENTION

The present invention is directed to compounds of formula (I) 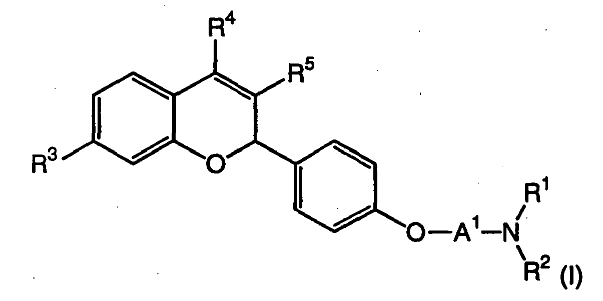 wherein
R¹ and R² are each independently selected from the group consisting of hydrogen and C₁₋₄alkyl;
alternatively, R¹ and R² are taken together with the nitrogen atom to which they are bound to form a 5 to 7 membered ring selected from the group consisting of heteroaryl and heterocycloalkyl; wherein the heteroaryl or heterocycloalkyl group is optionally substituted with one or more substituents independently selected from halogen, hydroxy, C₁₋₄alkyl, C₁₋₄alkoxy, carboxy, amino, C₁₋₄alkylamino, di(C₁₋₄alkyl)amino, nitro or cyano;
A¹ is -C₁₋₄alkyl-;
R³ is selected from the group consisting of hydroxy, C₁₋₄alkoxy, -O-Si(CH₃)₃ and -O-Si(t-butyl)(CH₃)₂;
R⁴ is selected from the group consisting of C₁₋₄alkyl, -C₁₋₄alkyl-OH, -C₁₋₄ alkyl-O-Si(CH₃)₃, -O-Si(t-butyl)(CH₃)₂, C₅₋₇cycloalkyl and C₅₋₇cycloalkenyl;
R⁵ is selected from the group consisting of hydrogen, C₁₋₄alkyl, -C₁₋₄ alkyl-OH, -C₁₋₄alkyl-O-Si(CH₃)₃, -O-Si(t-butyl)(CH₃)₂ and -CH₂-O-CH₂CH₂-Si(CH₃)₃;
alternatively, R⁴ and R⁵ are taken together to form a ring structure
selected from 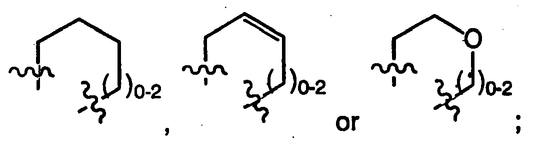 or a pharmaceutically acceptable salt thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Exemplifying the invention is the use of compound of formula (I) in the preparation of a medicament for treating a disorder mediated by one or more estrogen receptors in a subject in need thereof comprising administering to the subject a therapeutically effective amount of any of the compounds or pharmaceutical compositions described above.

Illustrating the invention is the use of compound of formula (I) in the preparation of a medicament for contraception comprising administering to a subject in need thereof co-therapy with a therapeutically effective amount of a compound of formula (I) with a progestogen or progestogen antagonist.

Another example of the invention is the use of any of the compounds described herein in the preparation of a medicament for treating: (a) hot flashes, (b) vaginal dryness, (c) osteopenia, (d) osteoporosis, (e) hyperlipidemia, (f) loss of cognitive function, (g) a degenerative brain disorder, (h) cardiovascular disease, (i) cerebrovascular disease (j) breast cancer, (k) endometrial cancer, (I) cervical cancer, (m) prostate-cancer, (n) benign prostatic hyperplasia, (o) endometriosis, (p) uterine fibroids, (q) osteoarthritis and for (r) contraception in a subject in need thereof.

### DETAILED DESCRIPTION OF THE INVENTION

The present invention is directed to compounds of formula (I) 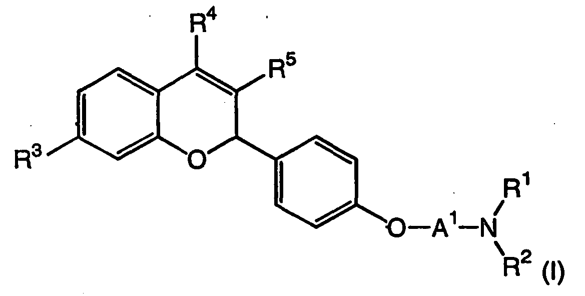 wherein R¹, R², R³, R⁴, R⁵ and A¹ are as herein defined. The compounds of the present invention are modulators of an estrogen receptor, useful for the treatment and prevention of disorders associated with estrogen depletion, including, but not limited to hot flashes, vaginal dryness, osteopenia, osteoporosis, hyperlipidemia, loss of cognitive function, degenerative brain diseases, cardiovascular and cerebrovascular diseases); for the treatment of hormone sensitive cancers and hyperplasia (in tissues including breast, endometrium, and cervix in women and prostate in men); for the treatment and prevention of endometriosis, uterine fibroids, and osteoarthritis; and as contraceptive agents either alone or in combination with a progestogen or progestogen antagonist.

In an embodiment of the present invention, the estrogen receptor is the α estrogen receptor. In another embodiment of the present invention, the estrogen receptor is the β estrogen receptor.

In an embodiment of the present invention, R¹ is selected from the group consisting of hydrogen and C₁₋₂alkyl. In another embodiment of the present invention, R¹ is C₁₋₂alkyl. Preferably, R¹ is methyl.

In an embodiment of the present invention, R² is selected from the group consisting of hydrogen and C₁₋₂alkyl. In another embodiment of the present invention, R² is C₁₋₂alkyl. Preferably, R² is methyl.

In an embodiment of the present invention, R¹ and R² are taken together with the nitrogen atom to which they are bound to form a 5 to 7 membered heteroaryl or a 5 to 7 membered saturated heterocycloalkyl group. In another embodiment of the present invention, R¹ and R² are taken together with the nitrogen atom to which they are bound to form a 5 to 7 membered saturated heterocycloalkyl group. Preferably, R¹ and R² are taken together with the nitrogen atom to which they are bound to form a group selected from piperidinyl or azepinyl.

In an embodiment of the present invention, R³ is selected from the group consisting of hydroxy, C₁₋₂alkoxy and -O-Si(t-butyl(CH₃)₂. In another embodiment of the present invention, R³ is selected from the group consisting of hydroxy and C₁₋₂alkoxy. In yet another embodiment of the present invention, R³ is selected from the group consisting of hydroxy and -O-Si(t-butyl(CH₃)₂. Preferably, R³ is hydroxy.

In an embodiment of the present invention, A¹ is -C₁₋₄alkyl-. In another embodiment of the present invention, A¹ is -C₁₋₃alkyl-. Preferably, A¹ is -CH₂-CH₂-.

In an embodiment of the present invention, R⁴ is selected from the group consisting of -C₁₋₄alkyl, -C₁₋₄alkyl-OH, C₅₋₇cycloalkyl and C₅₋₇cycloakenyl. In another embodiment of the present invention, R⁴ is selected from the group consisting of - C₁₋₂alkyl, -C₁₋₂alkyl-OH and C₅₋₆cycloalkyl. Preferably, R⁴ is selected from the group consisting of -CH₃, -CH₂CH₂-OH and cyclopentyl.

In an embodiment of the present invention, R⁵ is selected from the group consisting of hydrogen, -C₁₋₄alkyl, -C₁₋₄alkyl-OH and-CH₂-O-CH₂CH₂-Si(CH₃)₃. In another embodiment of the present invention, R⁵ is selected from the group consisting of hydrogen, -C₁₋₂alkyl-OH and-CH₂-O-CH₂CH₂-Si(CH₃)₃. Preferably, R⁵ is selected from the group consisting of hydrogen, -CH₂CH₂-OH and -CH₂-O-CH₂-CH₂-Si(CH₃)₃.

In an embodiment of the present invention, R⁴ and R⁵ are taken together to form a ring structure selected from 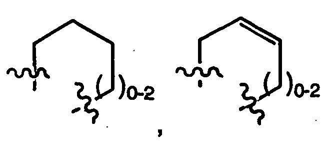 or 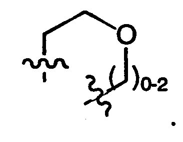 In another embodiment of the present invention, R⁴ and R⁵ are taken together to form a ring structure selected from 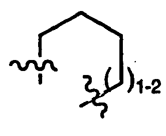 or 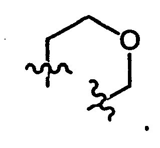 Preferably, R⁴ and R⁵ are taken together to form a ring structure selected from

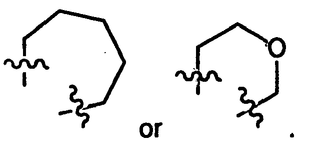

Additional embodiments of the present invention, include those wherein the substituents selected for one or more of the variables defined herein (i.e. R¹, R², R³, R⁴, R⁵ and A¹) are independently selected to be any individual substituent or any subset of substituents selected from the complete list as defined herein. In another embodiment of the present invention, are compounds selected from those listed in Table 1, below.

Representative compounds of the present invention are as listed in Table 1 below. Unless otherwise noted, the compounds were prepared as mixtures of stereo-configuration (where applicable). Where a stereogenic center is present, the S* and R* designations are intended to indicate that the exact stereo-configuration of the center has not been determined.

**Table 1**

| **ID No.** | **Structure** |
|---|---|
| 1 | 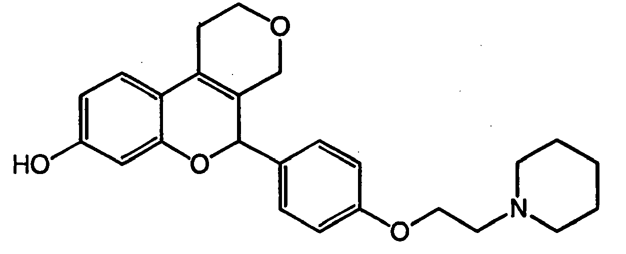 |
| 2 | 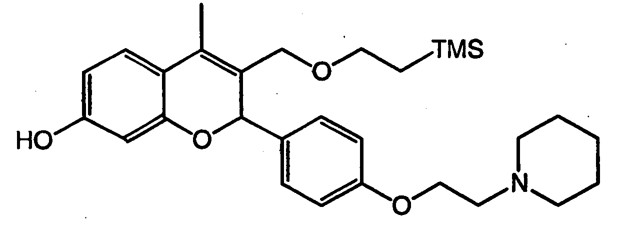 |
| 3 | 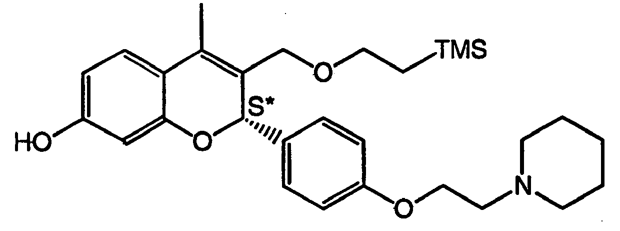 |
| 4 | 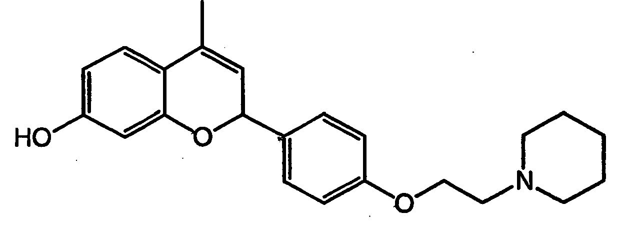 |
| 5 | 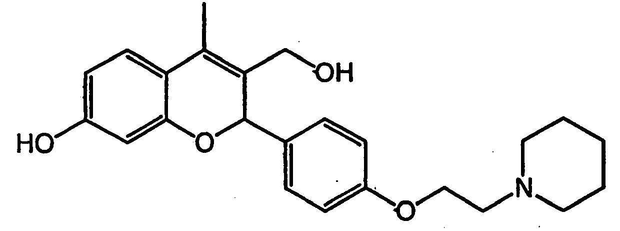 |
| 6 | 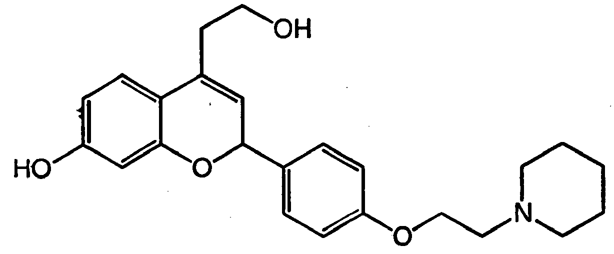 |
| 7 | 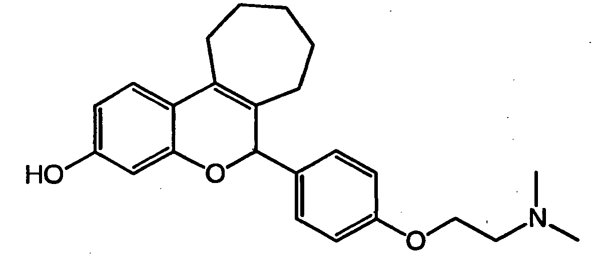 |
| 8 | 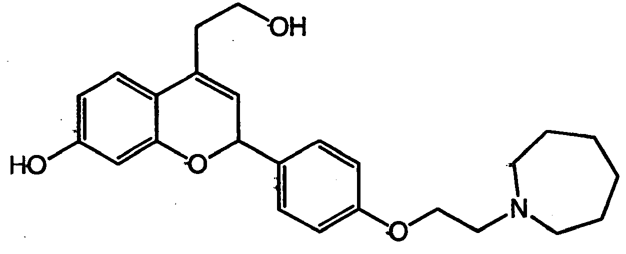 |
| 9 | 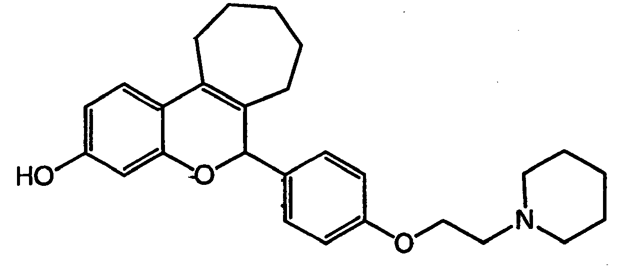 |
| 10 | 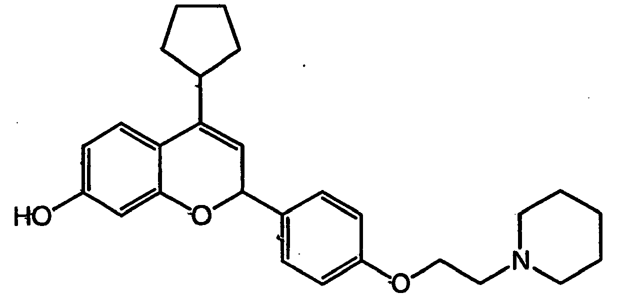 |
| 11 | 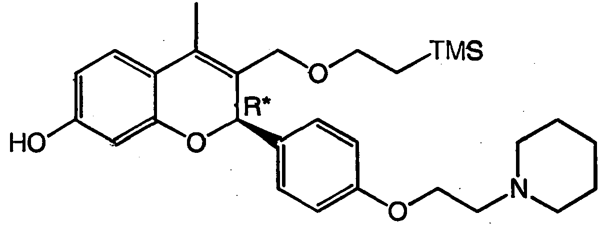 |
| 12 | 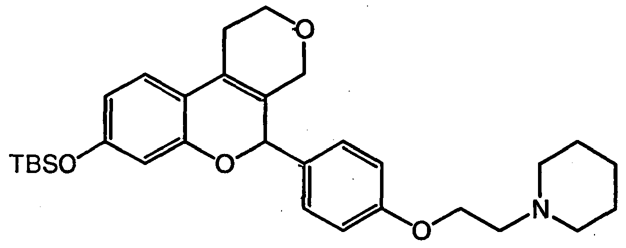 |

As used herein, the term "degenerative brain disease" shall include cognitive disorder, dementia, regardless of underlying cause, Alzheimer's disease, and the like.

As used herein, the term "cardiovascular disease" shall include elevated blood lipid levels, coronary arthrosclerosis, coronary heart disease, and the like.

As used herein, the term **"cerebrovascular disease"** shall include abnormal regional cerebral blood flow, ischemic brain damage, and the like.

As used herein, the term **"progestogen antagonist"** shall include mifepristone (RU-486), J-867 (Jenapharm / TAP Pharmaceuticals), J-956 (Jenapharm /TAP Pharmaceuticals), ORG-31710 (Organon), ORG-33628 (Organon), ORG-31806 (Organon), onapristone (ZK98299) and PRA248 (Wyeth). Further, as used herein, the terms **"progestin"** and **"progesterone"** are used interchangeably.

Estrogen receptor modulators are useful in the treatment and /or prevention of disorders and diseases mediated by one or more estrogen receptor(s) including, but not limited to, as hot flashes, vaginal dryness, osteopenia, osteoporosis, hyperlipidemia, loss of cognitive function, degenerative brain diseases, cardiovascular diseases, cerebrovascular diseases, hormone sensitive cancers, hyperplasia (in tissues including breast, endometrium, and cervix in women and prostate in men), endometriosis, uterine fibroids and osteoarthritis. Estrogen receptor modulators are further useful as contraceptive agents either alone or in combination with a progestogen or progestogen antagonist.
Preferably, the disorders and / or disease mediated by at least one estrogen receptor are selected from the group consisting of osteoporosis, hot flashes, vaginal dryness, breast cancer and endometriosis

As used herein, **"halogen"** shall mean chlorine, bromine, fluorine and iodine.

As used herein, the term **"alkyl"** whether used alone or as part of a substituent group, include straight and branched chains. For example, alkyl radicals include methyl, ethyl, n-propyl, isopropyl, n-butyl, isobutyl, sec-butyl, t-butyl, pentyl and the like. Similarly, the term **"C₁₋₄alkyl"** whether used alone or as part of a substituent group, include straight and branched chains containing 4 carbon atoms. For example, methyl, ethyl, n-propyl, isopropyl, n-butyl, isobutyl, sec-butyl and t-butyl.

As used herein, unless otherwise noted, **"alkoxy"** whether used alone or as part of a substituent group, shall denote an oxygen ether radical of the above described straight or branched chain alkyl groups. For example, methoxy, ethoxy, n-propoxy, sec-butoxy, t-butoxy, n-hexyloxy and the like. Similarly, the term **"C₁₋₄alkoxy"** whether used alone or as part of a substituent group, shall denote an oxygen ether radical of the above described straight or branched chain C₁₋₄alkyl groups. For example, methoxy, ethoxy, n-propoxy, sec-butoxy, t-butoxy, and the like.

As used herein, unless otherwise noted, the term **"C₅₋₇cycloalkyl"** shall denote any five to seven membered, saturated carbocyclic ring structure. Suitable example include, cyclopentyl, cyclohexyl and cycloheptyl. Similarly, unless otherwise noted, he term "C₅₋₇cycloalkenyl" shall denote any five to seven membered, partially unsaturated carbocyclic ring structure, wherein the ring structure contains at least one double bond, preferably one double bond. Suitable example include, cyclopentenyl, cycloohexenyl, cycloheptenyl, and the like.

As used herein, unless otherwise noted, the term **"partially unsaturated"** when referring to a ring structure shall mean any stable ring structure which contains at east one unsaturated bond. Suitable examples include, but are not limited to cyclohexenyl, and the like.

As used herein, unless otherwise noted, **"heteroaryl"** shall denote any five to seven membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. The heteroaryl group may be attached at any heteroatom or carbon atom of the ring such that the result is a stable structure.

Examples of suitable heteroaryl groups include, but are not limited to, pyrrolyl, furyl, thienyl, oxazolyl, imidazolyl, purazolyl, isoxazolyl, isothiazolyl, triazolyl, thiadiazolyl, pyridyl, pyridazinyl, pyrimidinyl, pyrazinyl, pyranyl, furazanyl, indolizinyl, indolyl, isoindolinyl, indazolyl, benzofuryl, benzothienyl, benzimidazolyl, benzthiazolyl, purinyl, quinolizinyl, quinolinyl, isoquinolinyl, isothiazolyl, cinnolinyl, phthalazinyl, quinazolinyl, quinoxalinyl, naphthyridinyl, pteridinyl, and the like. Preferred heteroaryl groups include pyrrolyl, pyridyl, pyrazolyl, pyrazinyl, and the like.

As used herein, unless otherwise noted, the term **"heterocycloalkyl"** shall denote any five to seven membered monocyclic, saturated or partially unsaturated ring structure containing at least one heteroatom selected from the group consisting of O, N and S, optionally containing one to three additional heteroatoms independently selected from the group consisting of O, N and S; or a nine to ten membered saturated, partially unsaturated or partially aromatic bicyclic ring system containing at least one heteroatom selected from the group consisting of O, N and S, optionally containing one to four additional heteroatoms independently selected from the group consisting of O, N and S. The heterocycloalkyl group may be attached at any heteroatom or carbon atom of the ring such that the result is a stable structure.
Examples of suitable heterocycloalkyl groups include, but are not limited to, pyrrolinyl, pyrrolidinyl, dioxalanyl, imidazolinyl, imidazolidinyl, pyrazolinyl, pyrazolidinyl, piperidinyl, dioxanyl, morpholinyl, dithianyl, thiomorpholinyl, piperazinyl, trithianyl, indolinyl, chromenyl, 3,4-methylenedioxyphenyl, 2,3-dihydrobenzofuryl, and the like. Preferred heterocycloalkyl groups include piperidinyl, morpholinyl, and the like.

As used herein, the notation "*" shall denote the presence of a stereogenic center.

When a particular group is "substituted" (e.g., aryl, heterocycloalkyl, heteroaryl), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

As used herein, unless otherwise noted, the term **"leaving group"** shall mean a charged or uncharged atom or group which departs during a substitution or displacement reaction. Suitable examples include, but are not limited to, Br, Cl, I, mesylate, tosylate, and the like.

Under standard nomenclature used throughout this disclosure, the terminal portion of the designated side chain is described first, followed by the adjacent functionality toward the point of attachment. Thus, for example, a "phenyl-alkylamino-carbonyl-alkyl" substituent refers to a group of the formula

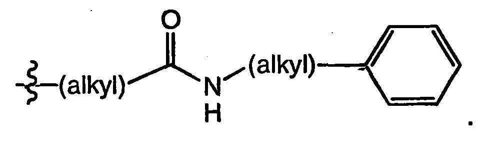

Abbreviations used in the specification, particularly the Schemes and Examples, are as follows:
- DCM: = Dichloromethane
- DEAD: = Diethyl azodicarboxylate
- Dibal-H or DIBAL-H: = Diisobutyl aluminum hydride
- DMF: = N,N-Dimethylformamide
- DTT: = Dithiothreitol
- ERT: = Estrogen replacement therapy
- Grubb's Catalyst: = Bis(tricyclohexylphosphine)benzylideneruthe nium (IV) dichloride
- HEPES: = 4-(2-Hydroxyethyl)-1-Piperizine Ethane Sulfonic Acid
- HPLC: = High Pressure Liquid Chromatography
- HRT: = Hormone replacement therapy
- DA: = Lithium Diisopropylamide
- LHMDS or LiHMDS: = Lithium Hexamethyldisilazinamide
- MOM: = Methoxy methyl
- MOM-Br: = Methoxy methyl bromide
- MOM-Cl: = Methoxy methyl chloride
- Pd-C or Pd/C: = Palladium on Carbon Catalyst
- SEM: = 2-(Trimethylsilyl)ethoxy methyl
- SEM-Cl: = 2-(Trimethylsilyl)ethoxy methyl chloride
- TBAF: = Tetra(n-butyl)ammonium fluoride
- TBS: = *Tert*-butyl-dimethyl-silyl
- TBSCI: = *Tert*-butyl-dimethyl-silyl chloride
- TEA or Et₃N: = Triethylamine
- TFA: = Trifluoroacetic acid
- THF: = Tetrahydrofuran
- TMS: = Trimethylsilyl
- TMSCI: = Trimethylsilyl chloride

The term **"subject"** as used herein, refers to an animal, preferably a mammal, most preferably a human, who has been the object of treatment, observation or experiment.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term **"composition"** is intended to encompass a product comprising the specified ingredients in the specified amounts, as well as any product which results, directly or indirectly, from combinations of the specified ingredients in the specified amounts.

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The present invention includes within its scope **prodrugs** of the compounds of this invention. In general, such prodrugs will be functional derivatives of the compounds which are readily convertible *in vivo* into the required compound. Thus, in the methods of treatment of the present invention, the term "administering" shall encompass the treatment of the various disorders described with the compound specifically disclosed or with a compound which may not be specifically disclosed, but which converts to the specified compound *in vivo* after administration to the patient. Conventional procedures for the selection and preparation of suitable prodrug derivatives are described, for example, in "Design of Prodrugs", ed. H. Bundgaard, Elsevier, 1985.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutically acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinc acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1 H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) may be prepared according to the process outlined in Scheme 1.

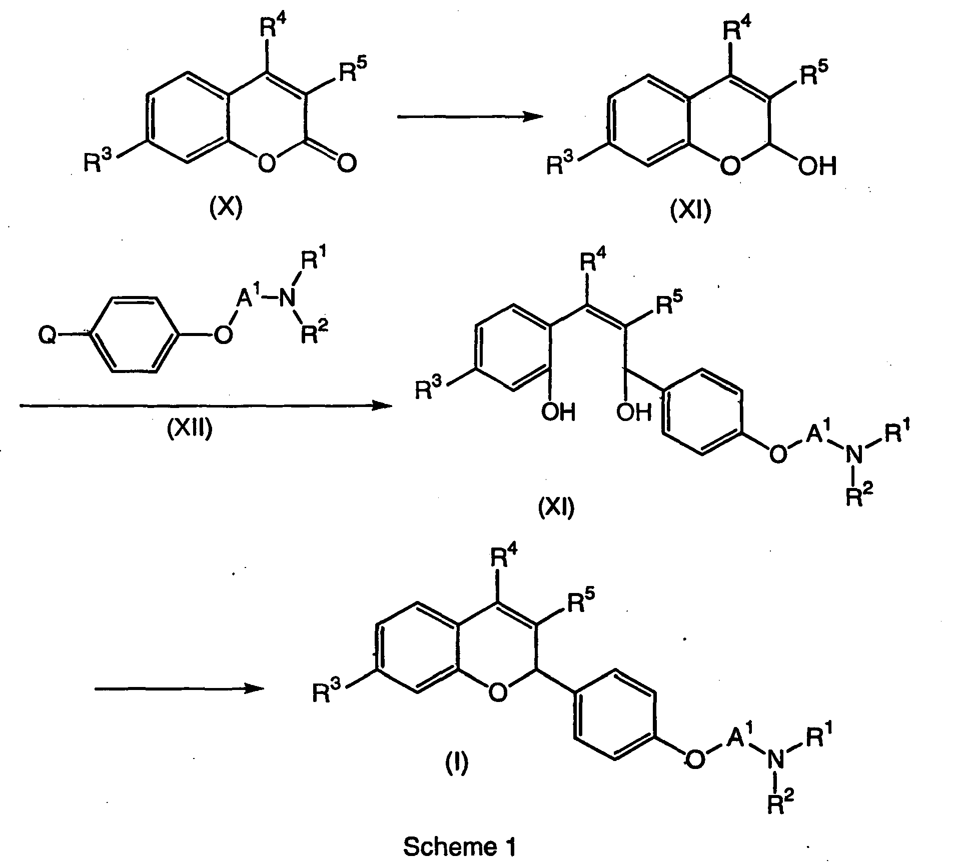

Accordingly, a suitably substituted compound of formula (X) is reduced according to known methods, for example, by reacting with Dibal-H in an organic solvent such as methylene chloride, THF, and the like, to yield the corresponding compound of formula (XI).

The compound of formula (XI) is reacted with a suitably substituted compound of formula (XII), wherein Q is Mg or Li, a known compound or compound prepared by known methods, according to known methods, to yield the corresponding compound of formula (XIII).

The compound of formula (XIII) is reacted with BF₃•Etherate, HCl, H₂SO₄, and the like, in an organic solvent such as methylene chloride, toluene, and the like, at a temperature in the range of from about 0°C to about room temperature, to yield the corresponding compound of formula (I).

Compounds of formula (X) are known compounds or compounds which may be prepared according to known methods. Schemes 2-7 below exemplify processes for the preparation of representative compounds of formula (X) and intermediates in their preparation. One skilled in the art will recognize that compounds of formula (X) and intermediates in their preparation which are not specifically described in the Schemes below may be similarly prepared by selecting and substituting suitably substituted starting materials and / or by protecting and de-protecting reaction sensitive groups (e.g. alkoxy groups).

As an example, intermediates in the synthesis of compounds of formula (X) may be prepared according to the process outlined in Scheme 2 below.

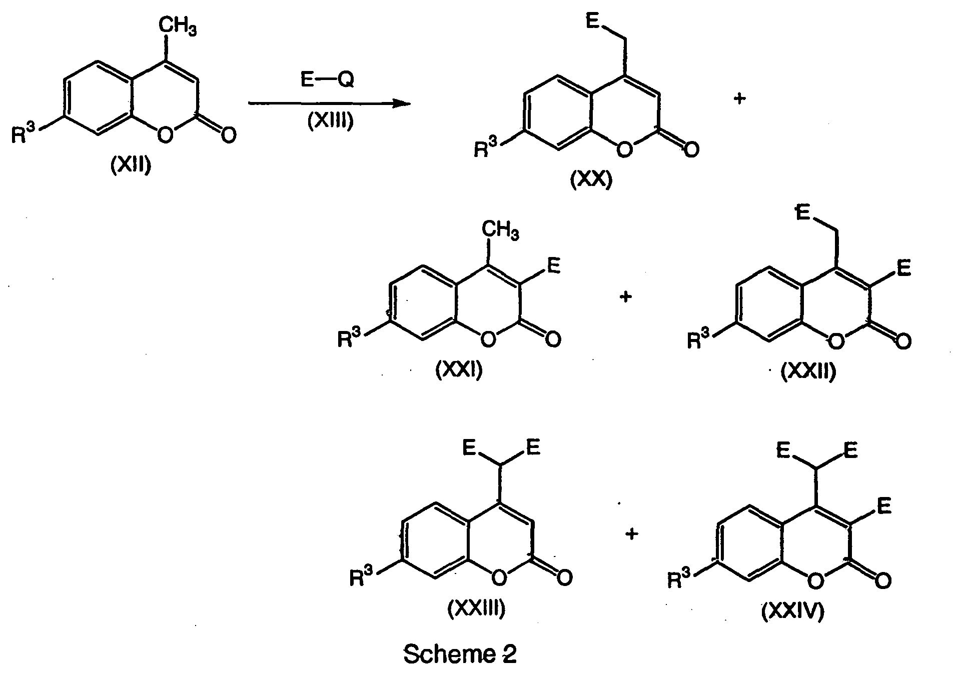

Accordingly, a suitably substituted compound of formula (XII), a known compound or compound prepared by known methods, is reacted with a base such as LDA, LiHMDS, and the like, and a compound of formula (XIII), where E-Q is SEM-Cl, MOM-Cl, allyl-Br, alkenyl-Br, benzyl-Br or methyl-I, and the like, a known compound or compound prepared by known methods, according to known methods, to yield a mixture of the corresponding compounds of formula (XX), (XXI), (XXII), (XXIII) and (XXIV). Preferably, the compounds of formula (XX), (XXI), (XXII), (XXIII) and (XXIV) are separated according to known methods, for example by column chromatography.

One skilled in the art will recognize that the ratio of the compounds of formula (XX), (XXI), (XXII), (XXIII) and (XXIV) within the mixture may be affected by controlling the temperature of the reaction and the amount or equivalents of the compound of formula (XIII) used.

Compounds of formula (X) wherein R³ is hydroxy, R⁶ is hydrogen and R⁴ is -CH₂-CH₂-OH may be prepared from the corresponding compound of formula (XX), according to the process outlined in Scheme 3, below.

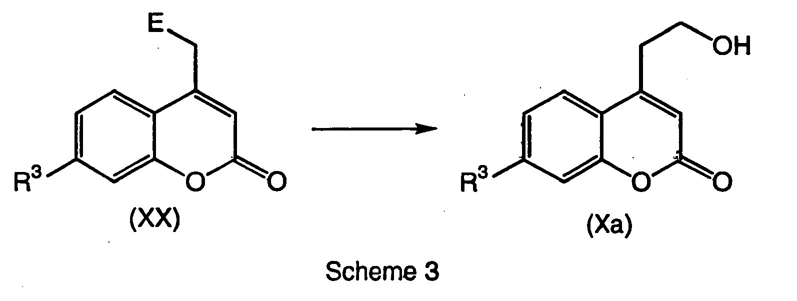

Accordingly, a suitably substituted compound of formula (XX) wherein E is SEM or MOM is reacted with BBr₃, in an organic solvent such as methylene chloride, chloroform, and the like, at a reduced temperature in the range of from about -78°C to about room temperature, to yield the corresponding compound of formula (Xa).

Compounds of formula (X) wherein R³ is hydroxy, R⁴ is methyl and R⁵ is -CH₂-OH or -CH₂-O-TMS may be prepared according to the process outlined in Scheme 4.

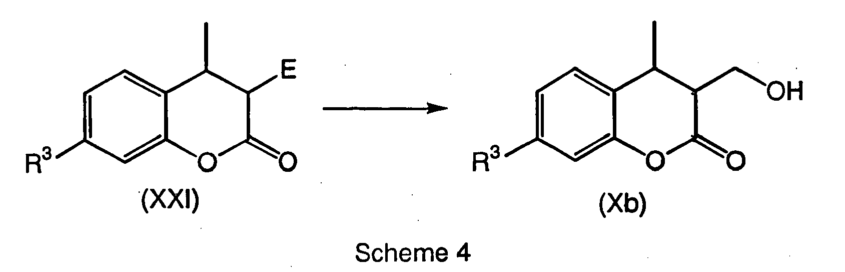

Accordingly, a suitably substituted compound of formula (XXII) is reacted with wherein E is SEM or MOM is reacted with BBr₃, in an organic solvent such as methylene chloride, chloroform, and the like, at a reduced temperature in the range of from about -78°C to about room temperature, to yield the corresponding compound of formula (Xb).

Compounds of formula (X) wherein R⁴ is C₅₋₇cycloalkyl or C₅₋₇ cycloalkenyl may be prepared according to the process outlined in Scheme 5.

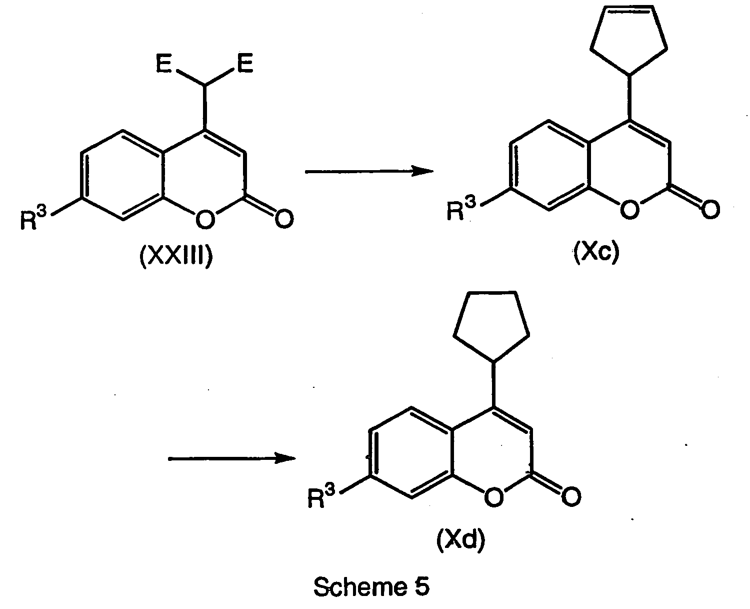

Accordingly, a suitably substituted compound of formula (XXIII), wherein E is -CH₂-CH=CH₂ is reacted with Grubb's catalyst, in a organic solvent such as THF, dioxane, and the like, at a temperature in the range of from about room temperature to about 60°C, to yield the corresponding compound of formula (Xc).

The compound of formula (Xc) is optionally hydrogenated, according to known methods, to yield the corresponding compound of formula (Xd).

One skilled in the art will recognize that compounds of formula (X) wherein R⁴ is C₆₋₇cycloalkyl or C₆₋₇cycloalkenyl may be similarly prepared according to the process outlined in Scheme 5 by reacting a compound of formula (XXIII) wherein E is -(CH₂)₁₋₂-CH=CH₂.

Compounds of formula (X) wherein R⁴ and R⁵ are taken together with the carbon atoms to which they are bound to form a 7 membered ring structure.

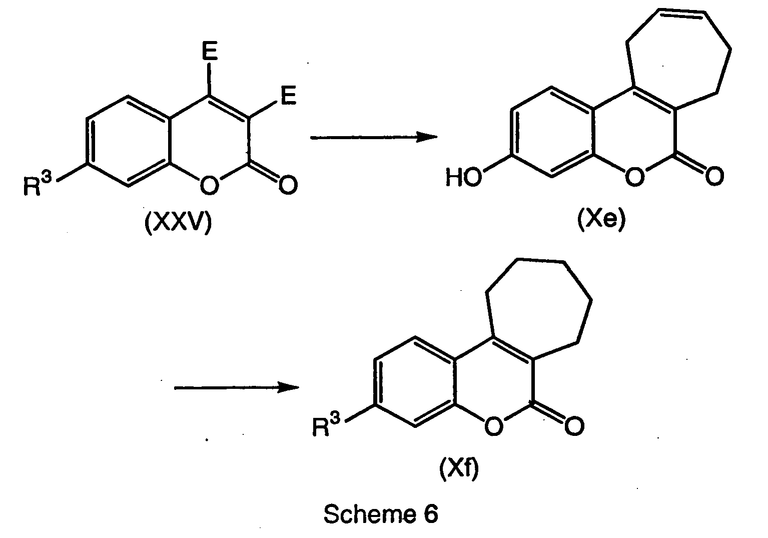

Accordingly, a suitably substituted compound of formula (XXV), wherein E is -CH₂-CH=CH₂, a known compound or compound prepared by known methods (for example by reacting an 7-R³ substituted 2H-chromene with E-Q, according to the method described in Scheme 2) is reacted with Grubb's catalyst, in a organic solvent such as THF, dioxane, and the like, at a temperature in the range of from about room temperature to about 60°C, to yield the corresponding compound of formula (Xe).

The compound of formula (Xe) is optionally hydrogenated, according to known methods, to yield the corresponding compound of formula (Xf).

Select compounds of formula (X) may be prepared according to the process outlined in Scheme 7.

### Step 1:

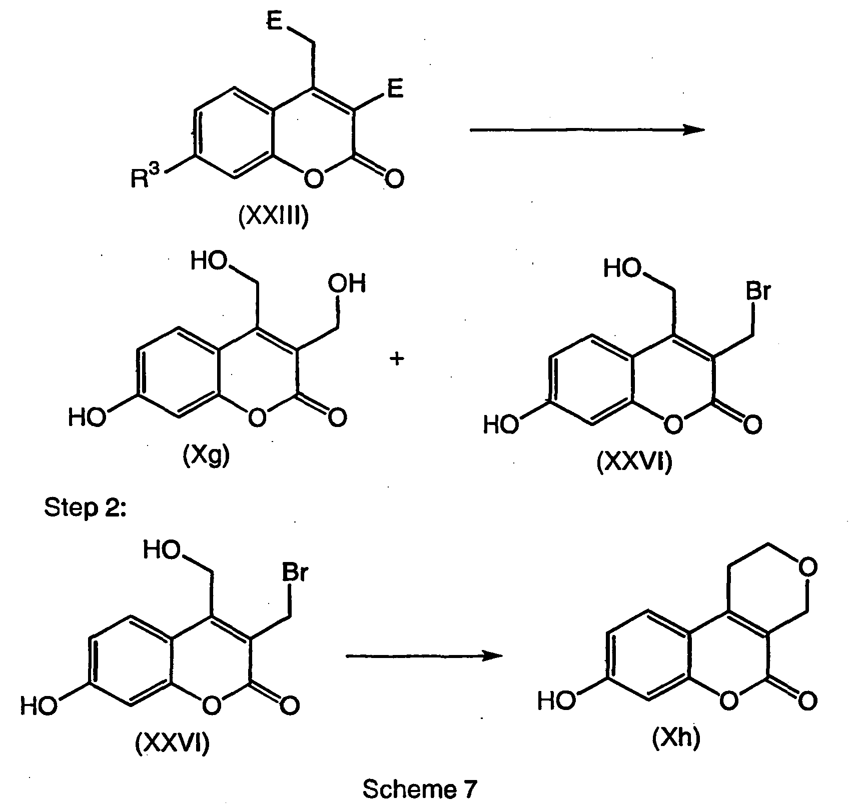

Accordingly, a suitably substituted compound of formula (XXII) is reacted with BBr₃ followed by treatment with a base such as NaOH, KOH, LiOH, and the like, in an organic solvent such as methanol, ethanol, and the like, or a mixture of an organic solvent and water such as methanol/water, and the like, to yield a mixture of the corresponding compound of formula (Xg) and (XXVI).

The mixture of the compounds of formula (Xg) and the compound of formula (XXVI) is separated according to known methods, for example by column chromatography.

The compounds of formula (XXVI) us reacted with a base such as NaOH, KOH, LiOH, NaH, LiHMDS, LDA, KHMDS, NaHMDS and the like, in solvent such as methanol, ethanol, THF, DMF water, and the like, to yield the corresponding compound of formula (Xh).

One skilled in the art will recognize that wherein the compound of formula (X) R⁴ and / or R⁵ is -C₁₋₄alkyl-OH, said terminal hydroxy group may be converted to the corresponding -C₁₋₄alkyl-OTMS by reacting the compound of formula (X) with TMSCI, according to known methods.
Similarly, one skilled in the art will recognize that compounds of formula (X) wherein R³ is -O-TMS or -OTBS may be prepared from the corresponding compound of formula (X) wherein R³ is hydroxy by reacting with a TMSCI or TBSCI, respectfully, according to known methods.

The present invention further comprises pharmaceutical compositions containing one or more compounds of formula (I) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more of the compounds of the present invention selected as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 50-100 mg and may be given at a dosage of from abut 0.5-5.0 mg/kg/day, preferably from about 1.0-3.0 mg/kg/day. The dosages, however, may be varied depending upon the requirement of the patients, the severity of the condition being treated and the compound being employed. The use of either daily administration or post-periodic dosing maybe employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The method of treating disorders mediated by one or more estrogen receptor described in the present invention may also be carried out using a pharmaceutical composition comprising any of the compounds as defined herein and a pharmaceutically acceptable carrier. The pharmaceutical composition may contain between about 0.01 mg and 1000 mg, preferably about 1 to 500 mg, more preferably, 10 to 100 mg of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover, when desired or necessary, suitable binders; lubricants, disintegrating agents and coloring agents can also be incorporated into the mixture. Suitable binders include, without limitation, starch, gelatin, natural sugars such as glucose or beta-lactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

The compound of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phophatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxy-ethylaspartamidephenol, or polyethyl eneoxidepolylysine substituted with palmitoyl residue. Furthermore, the compounds of the present invention may be coupled to a class of biodegradable polymers useful in achieving controlled release of a drug, for example, polylactic acid, polyepsilon caprolactone, polyhydroxy butyeric acid, polyorthoesters, polyacetals, polydihydropyrans, polycyanoacrylates and cross-linked or amphipathic block copolymers of hydrogels.

Compounds of this invention may be administered in any of the foregoing compositions and according to dosage regimens established in the art whenever treatment of disorders mediated by one or more estrogen receptors is required.

The daily dosage of the products may be varied over a wide range from 0.01 to 1,000 mg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250, 500 and 1000 milligrams of the active ingredient for the symptomatic adjustment of the dosage to the patient to be treated. An effective amount of the drug is ordinarily supplied at a dosage level of from about 0.01 mg/kg to about 300 mg/kg of body weight per day. Preferably, the range is from about 0.5 to about 5.0 mg/kg of body weight per day, most preferably, from about 1.0 to about 3.0 mg/kg of body weight per day. The compounds may be administered on a regimen of 1 to 4 times per day.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

The following Examples are set forth to aid in the understanding of the invention.

In the Examples which follow, some synthesis products are listed as having been isolated as a residue. It will be understood by one of ordinary skill in the art that the term "residue" does not limit the physical state in which the product was isolated and may include, for example, a solid, an oil, a foam, a gum, a syrup, and the like.

### Example 1

### 4-Methyl-2-[4-(2-piperidin-1-yl-ethoxy)-phenyl]-2H-chromen-7-ol (Compound ID #4)

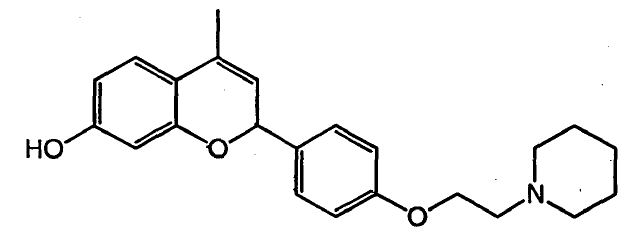

### STEP A: Preparation of 4-Methyl-7-(2-trimethylsilanyl-ethoxymethoxy)-2H-chromen-2-ol

To the solution of 4-methyl-7-(2-trimethylsilanyl-ethoxymethoxy)-chromen-2-one (1.53 g, 5 mmol) in toluene (50 mL) at -78°C was added slowly 1.5 M DIBAL-H in toluene (3.34 mL, 5 mmol). The reaction mixture was quenched with methanol (1 mL) after stirring at -78°C for 30 min. The reaction mixture was then diluted with ethyl acetate (500 mL) and washed with saturated aqueous sodium potassium tartarate solution (4 x 200 ml). The organic layer was dried over sodium sulfate and concentrated to yield a crude product as a colorless oil. The crude product was used in the next step without further purification.

### STEP B: Preparation of 4-Methyl-2-[4-(2-piperidin-1-yl-ethoxy)-phenyl]-2H-chromen-7-ol

To a solution of 1-[2-(4-iodo-phenoxy)-ethyl]-piperidine (15 mmol) (5.0 g) in THF (30 mL) at -78^{°}C was added dropwise n-butyllithium (6.0 mL, 2.5 M in hexane). The reaction mixture was then stirred at -78°C for 30 min. To the reaction mixture was then slowly added a solution of the colorless oil prepared in Step A above in THF (20 mL). The reaction mixture was stirred for another 30 min after the addition and then quenched with aqueous ammonium chloride. The reaction mixture was then extracted with ethyl acetate and the organic layer was combined and dried over sodium sulfate and concentrated to yield a crude product. The crude product was dissolved in toluene (250 mL) and then 36.5% HCl (1.7 mL) was added. The solution was stirred for 30 min and neutralized with 5% NaHCO₃. The mixture was extracted with a THF-ethyl acetate mixture and the organic layer was combined, dried over sodium sulfate and concentrated to yield crude product. The crude product was dissolved into acetonitrile (30 mL) and then 70% HF in pyridine (2mL) was added, at room temperature. The reaction mixture was stirred overnight and diluted with ethyl acetate and washed with 5% NaHCO₃, then with brine. The organic layer was dried over sodium sulfate and concentrated. The crude product was purified with flash column chromatography eluting with 2% methanol in dichloromethane. The product was further purified with HPLC to yield the title compound as a red solid.
LCMS: 4.831 min, 366 (M + 1);
¹HNMR (CDCl₃, 400 MHz) δ (ppm) 7.3 (d, J = 8.4 Hz, 2H), 7.05 (d, *J* = 8.4 Hz, 1 H), 6.8 (d, *J* = 8.4 Hz, 2H), 6.4 (dd, *¹J* = 8.4 Hz, *²J* = 2 Hz, 1 H), 6.3 (d, *J* = 2 Hz, 1 H), 5.75 (s, 1 H), 5.45 (s, 1 H), 4.1 (t, *J* = 6 Hz, 2H), 2.8 (t, *J* = 6 Hz, 2H), 2.55 (bs, 4H), 2.05 (s, 3H), 1.65 (m, 4H), 1.45 (m, 2H).

### Example 2

### 4-Methyl-2-[4-(2-piperidin-1-yl-ethoxy)-phenyl]-3-(2-trimethylsilanyl-ethoxymethyl)-2H-chromen-7-ol (Compound ID #2)

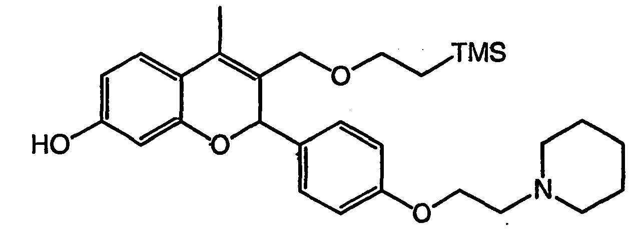

### STEP A: Preparation of 4-Methyl-7-(2-trimethylsilanyl-ethoxymethoxy)-3-(2-trimethylsilanyl-ethoxymethyl)-chromen-2-one

To a solution of 4-methyl-7-(2-trimethylsilanyl-ethoxymthoxy)-chromen-2-one (306 mg, 1 mmol) in THF (10 mL) at -10°C was added LiHMDS (1.5 mL, 1.0 M in THF). The resulting slight yellow solution was stirred for 30 min before the addition of SEMCI (0.21 mL, 1.2 mmol). After 2 hours the reaction was quenched with aqueous ammonium chloride and extracted with ethyl acetate. The organic layer was dried over sodium sulfate and concentrated. Flash column yielded a colorless oil of 4-methyl-7-(2-trimethylsilanyl-ethoxymethoxy)-3-(2-trimethylsilanyl-ethoxymethyl)-chromen-2-one.
¹HNMR (CDCl₃, 400 MHz) δ (ppm) 7.8 (d, *J* = 8.4 Hz, 1H), 7.2 (d, *J* = 8.4 Hz, 2H), 5.51 (s, 2H), 4.77 (s, 2H), 4.01 (t, *J* = 8.4 Hz, 2H), 3.87 (t, *J* = 8.4 Hz, 2H), 2.73 (s, 3H), 1.22 (m, 4H), 0.26 (s, 9H), 0.24 (s, 9H);
¹³CNMR (CDCl₃, 100.6 MHz) δ (ppm) 161.9, 160.4, 154.4, 151.4, 126.0, 119.9, 115.0, 113.4, 103.7, 93.1, 68.2, 66.9, 63.9, 18.5, 18.5, 18.2, 15.1;
MS: 459 (M + 23)

### STEP B: Preparation of 4-Methyl-7-(2-trimethylsilanyl-ethoxymethoxy)-3-(2-trimethylsilanyl-ethoxymethyl)-2H-chromen-2-ol

To a solution of 4-methyl-7-(2-trimethylsilanyl-ethoxymethoxy)-3-(2-trimethylsilanyl-ethoxymethyl)-chromen-2-one (605 mg, 1.39 mmol) in toluene (15 mL) at -15^{°}C was added slowly 1.5 M DIBAL-H in toluene (1.0 mL). The reaction was quenched with sodium potassium tartarate aqueous solution at - 78^{°}C. The reaction mixture was then diluted with ethyl acetate, washed with sodium potassium tartarate aqueous solution twice. The aqueous layer was extracted with ethyl acetate once. The combined organic layer was dried over sodium sulfate, then concentrated and purified on silica gel column to yield a colorless oil of 4-methyl-7-(2-trimethylsilanyl-ethoxymethoxy)-3-(2-trimethylsilanyl-ethoxymethyl)-2H-chromen-2-ol.
¹HNMR (CDCl₃, 400 MHz) δ (ppm) 7.77 (d, *J* = 8.4 Hz, 1H), 7.24 (m, 2H), 6.45 (d, *J* = 6.8 Hz, 1 H), 5.73 (s, 2H), 4.77 (s, 2H), 4.27 (t, *J* = 8.4 Hz, 2H), 4.08 (m, 2H), 3.99 (d, *J* = 6.8 Hz, 1 H), 2.65 (s, 3H), 1.50 (m, 4H), 0.55 (s, 9H), 0.53 (s, 9H);
MS: 461 (M + 23)

### STEP C: Preparation of 2-[3-Hydroxy-1-methyl-3-[4-(2-piperidin-1-yl-ethoxy)-phenyl]-2-(2-trimethylsilanyl-ethoxymethyl)-propenyl]-5-(2-trimethylsilanyl-ethoxymethoxy)-phenol

To the solution of 1-[2-(4-iodo-phenoxy)-ethyl]-piperidine (894 mg, 2.7 mmol) in THF (5 mL) at -78^{°}C was added dropwise n-butyllithium (1.1 mL, 2.5 M in hexane). The solution was stirred at -78^{°}C for 30 min, then a solution of 4-methyl-7-(2-trimethylsilanyl-ethoxymethoxy)-3-(2-trimethylsilanyl-ethoxymethyl)-2H-chromen-2-ol (395 mg, 0.9mmol) in THF (4 mL) was added slowly. The reaction mixture was stirred for another 30 min, then quenched with aqueous ammonium chloride and extracted with ethyl acetate. The organic layer was combined and dried over sodium sulfate, then concentrated to yield a crude product which was used in the next step without further purification.
LCMS: 5.810 min, 644 (M + 1).

### STEP D: Preparation of 1-(2-{4-[4-Methyl-7-(2-trimethylsilanyl-ethoxymethoxy)-3-(2-trimethylsilanyl-ethoxymethyl)-2H-chromen-2-yl]-phenoxy}-ethyl)-piperidine

The crude product prepared in Step C above, was dissolved into toluene (50 mL) and cooled to 0^{°}C. To the reaction mixture was then added 36.5% HCl (0.3 mL). The solution was stirred for 30 min, neutralized with 5% NaHCO₃ and extracted with THF-ethyl acetate mixture. The organic layer was combined and dried over sodium sulfate, concentrated and the residue purified by flash column with 2:98:0.3 MeOH: CH₂Cl₂: TEA to yield 1-(2-{4-[4-Methyl-7-(2-trimethylsilanyl-ethoxymethoxy)-3-(2-trimethylsilanyl-ethoxymethyl)-2H-chromen-2-yl]-phenoxy}-ethyl)-piperidine as an oil.

### STEP E: Preparation of 4-Methyl-2-[4-(2-piperidin-1-yl-ethoxy)-phenyl]-3-(2-trimethylsilanyl-ethoxymethyl)-2H-chromen-7-ol

To a solution of 1-(2-{4-[4-Methyl-7-(2-trimethylsilanyl-ethoxymethoxy)-3-(2-trimethylsilanyl-ethoxymethyl)-2H-chromen-2-yl]-phenoxy}-ethyl)-piperidine (453 mg, 0.72 mmol) in THF (7 mL) was added 1.0 M TBAF in THF (1.8 mL) at room temperature. The reaction mixture was stirred overnight and additional of 1.0 M TBAF in THF (2 mL) was added. The reaction mixture was then refluxed for 4 hours. The resulting mixture was diluted with ethyl acetate, washed with ammonium chloride, then with brine. The organic layer was dried over sodium sulfate and concentrated. The crude product was purified with flash column chromatography eluting with 2% Methanol in dichloromethane to yield 4-Methyl-2-[4-(2-piperidin-1-yl-ethoxy)-phenyl]-3-(2-trimethylsilanyl-ethoxymethyl)-2H-chromen-7-ol as an oil.
HPLC: 5.094 min
¹HNMR (CDCl₃, 400 MHz) δ (ppm) 7.2 (d, *J* = 8.4 Hz, 2H), 7.05 (d, *J* = 8.4 Hz, 1 H), 6.65 (d, *J* = 6.8 Hz, 2H), 6.35 (dd, *¹J* = 8.4 Hz, *²J* = 2 Hz, 1 H), 6.20 (d, *J* = 2 Hz, 1 H), 5.75 (s, 1H), 4.2 (d, *J* = 10 Hz, 1 H), 4.0 (t, *J* = 6 Hz, 2H), 3.7 (d, *J* = 10 Hz, 1 H), 3.53 (m, 1 H), 3.35 (m, 1H), 2.75 (m, 2H), 2.55 (bs, 4H), 2.1 (s, 3H), 1.6 (m, 4H), 1.45 (m, 2H), 0.9 (m, 2H), 0 (s, 9H);

### Example 3

### S*-4-Methyl-2-[4-(2-piperidin-1-yl-ethoxy)-phenyl]-3-(2-trimethylsilanyl ethoxymethyl)-2H-chromen-7-ol and R*-4-Methyl-2-[4-(2-piperidin-1-yl-ethoxy)-phenyl]-3-(2-trimethylsilanyl ethoxymethyl)-2H-chromen-7-ol (Compound ID #3 and Compound ID #11)

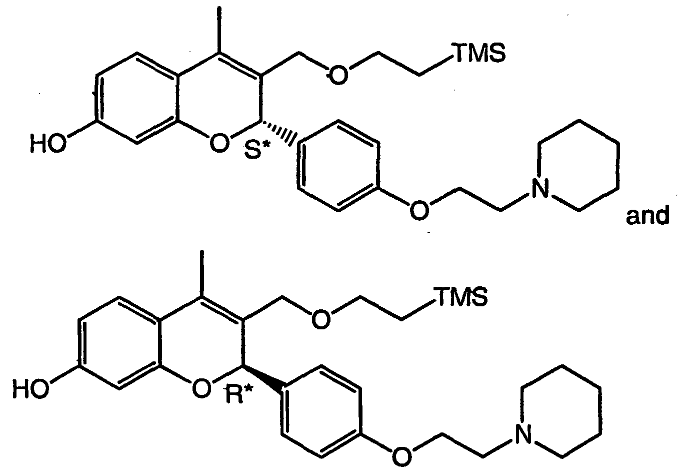

The racemic compound 4-methyl-2-[4-(2-piperidin-1-yl-ethoxy)-phenyl]-3-(2-trimethylsilanyl ethoxymethyl)-2H-chromen-7-ol (2.5g) was loaded onto a ChiralPak AD chiral HPLC column (5 cm I.D. x 50 cm L) and eluted with 20%MeOH in IPA at the 90 mL/min flow rate. The two peaks were removed under vacuum to yield as follows:

### Peak 1: S*- 4-Methyl-2-[4-(2-piperidin-1-yl-ethoxy)-phenyl]-3-(2-trimethylsilanyl ethoxymethyl)-2H-chromen-7-ol

¹HNMR (CDCl₃, 400 MHz) δ (ppm) 7.2 (d, *J* = 8.4 Hz, 2H), 7.05 (d, *J* = 8.4 Hz, 1H), 6.65 (d, *J* = 6.8 Hz, 2H), 6.35 (dd, *¹J* = 8.4 Hz, *²J* = 2 Hz, 1 H), 6.20 (d, *J* = 2 Hz, 1 H), 5.75 (s, 1 H), 4.2 (d, *J* = 10 Hz, 1 H), 4.0 (t, *J* = 6 Hz, 2H), 3.7 (d, *J* = 10 Hz, 1H), 3.53 (m, 1 H), 3.35 (m, 1 H), 2.75 (m, 2H), 2.55 (bs, 4H), 2.1 (s, 3H), 1.6 (m, 4H), 1.45 (m, 2H), 0.9 (m, 2H), 0 (s, 9H);
(α)_{D=} -12 ( C= 0.5, MeOH).

### Peak 2: R*- 4-Methyl-2-[4-(2-piperidin-1-yl-ethoxy)-phenyl]-3-(2-trimethylsilanyl ethoxymethyl)-2H-chromen-7-ol

(α)_{D=} +12 ( C= 0.5, MeOH).

### Preparative Example 4

### 7-(tert-Butyl-dimethyl-silanyloxy)-4-methyl-chromen-2-one

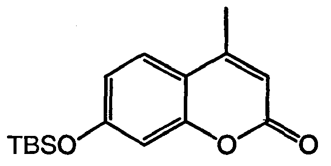

7-hydroxy-4-methylcoumarin (35.2 g, 0.20 mol) and t-butyldimethylsilyl chloride (30.0 g, 0.20 mol) were dissolved in CH₂Cl₂ (200 mL) at room temperature and then treated with TEA (28 mL, 0.20 mol). After 1 hour ethyl ether (600 mL) was poured into the solution and the reaction mixture was transferred into a separation funnel. The reaction mixture was washed with 5% sodium bicarbonate (2 x 250 ml), water (250 ml) and then with brine. The organic layer was dried over sodium sulfate and concentrated to yield the title compound a crude product, which was used in the next step without further purification.

### Preparative Example 5

### 7-(tert-Butyl-dimethyl-silanyloxy)-4-(2-methoxy-ethyl)-3-methoxymethyl-chromen-2-one and 7-(tert-Butyl-dimethyl-silanyloxy)-4-(2-methoxy-ethyl)-chromen-2-one

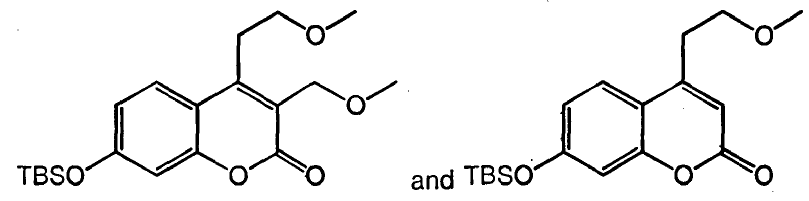

To a solution of 7-hydroxy-4-methylcoumarin (11.6 g, 40 mmol) in dry THF (200 mL) at - 20^{°}C was added dropwise 1.0 M LiHMDS solution in THF (60 mL). After 30 minutes, MOMBr (5.44 mL) was added slowly into the solution and the reaction mixture stirred for 1 hour. The reaction mixture was quenched with aqueous ammonium chloride and extracted with ethyl acetate. The organic layer was combined and dried over sodium sulfate, then concentrated and purified on silica gel eluting with 30% ethyl acetate in hexane to yield a colorless oil, which was a mixture of two compounds: 7-(tert-Butyl-dimethyl-silanyloxy)-4-(2-methoxy-ethyl)-3-methoxymethyl-chromen-2-one and 7-(tert-Butyl-dimethyl-silanyloxy)-4-(2-methoxy-ethyl)-chromen-2-one.

### Preparative Example 6

### 3-Bromomethyl-7-(tert-butyl-dimethyl-silanyloxy)-4-(2-hydroxy-ethyl)-chromen-2-one and 7-(tert-Butyl-dimethyl-silanyloxy)-4-(2-hydroxy-ethyl)-chromen-2-one

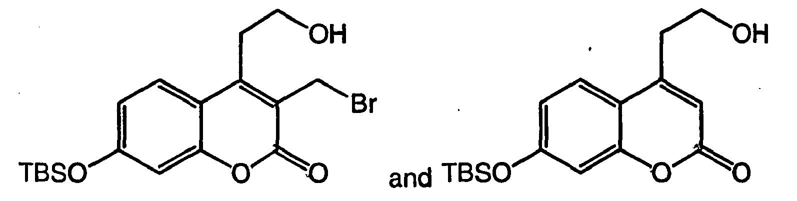

A mixture of 7-(tert-Butyl-dimethyl-silanyloxy)-4-(2-methoxy-ethyl)-3-methoxymethyl-chromen-2-one and 7-(tert-Butyl-dimethyl-silanyloxy)-4-(2-methoxy-ethyl)-chromen-2-one (7.48 g) was dissolved into DCM (100 mL) and cooled to - 78°C. Boron tribromide (4.3 mL) was then slowly added to the reaction mixture. The reaction mixture was then slowly warmed to room temperature and stirred for 4 hours. The reaction mixture was washed with 5% sodium bicarbonate and then with brine. The organic layer was dried over sodium sulfate and concentrated to yield a crude product which was purified by flash column chromatography on silica gel eluting with 50% ethyl acetate in hexane to yield 3-Bromomethyl-7-(tert-butyl-dimethyl-silanyloxy)-4-(2-hydroxyethyl)-chromen-2-one and 7-(tert-Butyl-dimethyl-silanyloxy)-4-(2-methoxyethyl)-chromen-2-one as an oil.

### Preparative Example 7

### 7-(tert-Butyl-dimethyl-silanyloxy)-4-[2-(tert-butyl-dimethyl-silanyloxy)-ethyl]-chromen-2-one

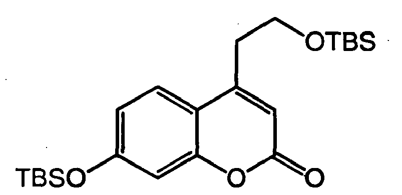

7-(tert-Butyl-dimethyl-silanyloxy)-4-(2-hydroxy-ethyl)-chromen-2-one (1.20 g, 3.74 mmol), t-butyldimethylsilyl chloride (565 mg, 3.74 mmol) and imidazole (255 mg, 3.74 mmol) were dissolved in DMF (10 mL) and the resulting mixture was stirred at room temperature for 2 hours. The reaction mixture was then diluted with ethyl ether, washed with 5% sodium bicarbonate and then with brine. The organic layer was dried over sodium sulfate, concentrated and purified by column chromatography to yield a colorless oil of 7-(tert-Butyl-dimethyl-silanyloxy)-4-[2-(tert-butyl-dimethyl-silanyloxy)-ethyl]-chromen-2-one.
¹HNMR (CDCl₃, 300 MHz) δ (ppm) 7.55 (d, *J* = 8.4 Hz, 1 H), 6.75 (dd, ¹*J* = 8.4 Hz, ²*J* = 2 Hz, 1 H), 6.75 (d, *J* = 2 Hz, 1 H), 6.2 (s, 1 H), 3.95 (t, *J* = 8.4 Hz, 2H), 2.95 (t, *J* = 8.4 Hz, 2H), 1.0 (s, 9H), 0.85 (s, 9H), 0.25 (s, 6H), 0 (s, 6H);
LCMS: 12.863 min; m/z, 435 (M + 1), 891 (2M + 23)

### Example 8

### 4-(2-Hydroxy-ethyl)-2-[4-(2-piperidin-1-yl-ethoxy)-phenyl]-2H-chromen-7-ol (Compound ID #6)

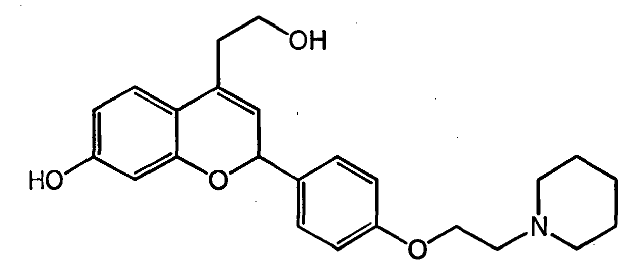

### STEP A: Preparation of 7-(tert-Butyl-dimethyl-silanyloxy)-4-[2-(tert-butyl-dimethyl-silanyloxy)-ethyl]-2H-chromen-2-ol

To a solution of 7-(tert-Butyl-dimethyl-silanyloxy)-4-[2-(tert-butyl-dimethyl-silanyloxy)-ethyl]-chromen-2-one (1.45 g, 3.34 mmol) in toluene (30 mL) at -78^{°}C was added slowly 1.5 M DIBAL-H in toluene (2.7 mL, 4.05 mmol). The reaction mixture was stirred at -78°C for 30 min, then quenched with aqueous ammonium chloride. The reaction mixture was then diluted with ethyl acetate and washed with saturated aqueous sodium potassium tartarate solution. The organic layer was dried over sodium sulfate and concentrated to yield a residue. The residue was azeotropic evaporated with benzene, dried under vacuum and used for the next step without further purification.

### STEP B: Preparation of 4-(2-Hydroxy-ethyl)-2-[4-(2-piperidin-1-yl-ethoxy)-phenyl]-2H-chromen-7-ol

1-[2-(4-iodo-phenoxy)-ethyl]-piperidine (3.3 g, 10 mmol) was dissolved in THF (20 mL) and cooled to -78^{°}C. To the solution was added dropwise n-butyllithium (4.0 mL, 2.5 M in hexane). The solution was stirred at -78°C for 30 min before the addition of the crude product prepared in Step A above in THF (10 mL). The reaction mixture was stirred for an additional 30 min after the addition, then quenched with aqueous ammonium chloride and extracted with ethyl acetate. The organic layer was combined, dried over sodium sulfate and concentrated to yield a crude product. The crude product was dissolved into toluene (100 mL) and then cooled to 0^{°}C. TFA (1 mL) was then added to the reaction mixture dropwise. The reaction mixture was stirred at 0°C for 1 hour, then diluted with ethyl acetate and washed with 5% NaHCO₃, then with brine. The organic layer was dried over sodium sulfate and concentrated to yield a crude product. The crude product was dissolved into acetonitrile (30 mL). To the reaction mixture was then added pyridine (2 mL) pyridine and 70% HF-pyridine (1 mL). The reaction mixture was stirred overnight at room temperature, then diluted with ethyl acetate and washed with aqueous 5% NaHCO₃, then with brine. The organic layer was combined, dried over sodium sulfate and concentrated to yield a crude product. The crude product was purified with flash column chromatography eluting with 5:95:1 methanol : dichloromethane : TEA to yield an orange solid of 4-(2-Hydroxy-ethyl)-2-[4-(2-piperidin-1-yl-ethoxy)-phenyl]-2H-chromen-7-ol.
¹HNMR (CD₃OD, 300 MHz) δ (ppm) 7.25 (d, *J* = 8.4 Hz, 2H), 7.05 (d, *J* = 8.4 Hz, 1H), 6.8 (d, *J* = 8.4 Hz, 2H), 6.3 (dd, *¹J* = 8.4 Hz, *²J*= 2 Hz, 1 H), 6.15 (d, *J* = 2 Hz, 1 H), 5.65 (d, *J* = 2 Hz, 1 H), 5.5 (d, *J* = 2 Hz, 1 H), 4.5 (m, 1 H), 4.1 (t, *J* = 6 Hz, 2H), 3.75 (t, *J* = 6 Hz, 1 H), 3.65 (m, 1 H), 2.95 (t, *J* = 6 Hz, 2H), 2.55 (m, 4H), 1.6 (m, 6H).

### Preparative Example 9

### 8-(tert-Butyl-dimethyl-silanyloxy)-1,4-dihydro-2H-pyrano[3,4-c]chromen-5-one

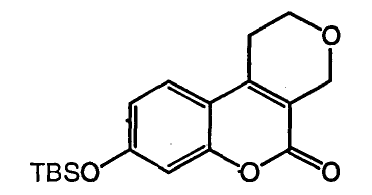

To a solution of 3-Bromomethyl-7-(tert-butyl-dimethyl-slianyloxy)-4-(2-hydroxy-ethyl)-chromen-2-one (2.0 g, 4.84 mmol) in THF at 0^{°}C was added dropwise 1 M LiHMDS in THF (5 mL). The reaction mixture was stirred for 1 hour, then quenched with aqueous ammonium chloride and extracted with ethyl acetate. The organic layer was dried over sodium sulfate and concentrated to yield a residue. Flash column chromatography purification of the residue yielded yellow crystals of 8-(tert-Butyl-dimethyl-silanyloxy)-1,4-dihydro-2H-pyrano[3,4-c]chromen-5-one.
¹HNMR (CDCl₃, 300 MHz) δ (ppm) 7.4 (d, *J* = 8.4 Hz, 1 H), 6.8 (m, 2H), 4.6 (t, *J* = 1 Hz, 2H), 4.0 (t, *J* = 6 Hz, 2H), 2.8 (m, 2H), 1.0 (s, 9H), 0.25 (s, 6H);
LCMS: 8.653 min, >97%; m/z, 333 (M + 1), 355 (M + 23), 687 (2M + 23);

### Preparative Example 10

### 8-(tert-Butyl-dimethyl-silanyloxy)-1,5-dihydro-3H,4H-pyrano[3,4-c]chromen-5-ol

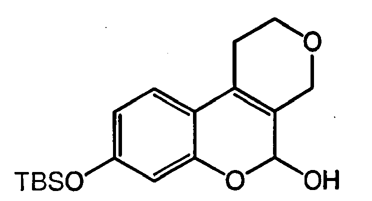

To a solution of 8-(tert-Butyl-dimethyl-silanyloxy)-1,4-dihydro-2H-pyrano[3,4-c]chromen-5-one (300 mg, 0.90 mmol) in toluene (10 mL) at -78°C was added slowly 1.5 M DIBAL-H in toluene (0.6 mL, 0.90 mmol). The reaction mixture was stirred at -78°C for 30 min., then quenched with aqueous ammonium chloride. The reaction mixture was then diluted with ethyl acetate and washed with saturated aqueous sodium potassium tartarate solution. The organic layer was dried over sodium sulfate and concentrated to yield a residue. The residue was azeotropic evaporated with benzene, then dried under vacuum to yield 8-(tert-Butyl-dimethyl-silanyloxy)-1,5-dihydro-2H,4H-pyrano[3,4-c]chromen-5-ol as a solid.
¹HNMR (CDCl₃, 400 MHz) δ (ppm) 7.05 (d, *J* = 8.4 Hz, 1 H), 6.5 (m, 2H), 5.7 (d, *J* = 8.4 Hz, 1 H), 4.4 (td, ¹*J* = 12 Hz, ²*J* = 1 Hz, 1H), 4.2 (td, ¹*J* = 12 Hz, ²*J* = 1 Hz,1H), 3.95 (m, 2H), 3.1 (d, *J* = 8.4 Hz, 1 H), 2.6 (m, 1 H), 2.4 (m, 1 H), 0.95 (s, 9H), 0.20 (s, 6H);
LCMS: 7.741 min, >97%; m/z, 317 (M -H₂O + 1)

### Example 11

### 1-(2-{4-[8-(tert-Butyl-dimethyl-silanyloxy)-1,5-dihydro-2H,4H-pyrano[3,4-c]chromen-5-yl]-phenoxy}-ethyl)-piperidine (Compound ID#12)

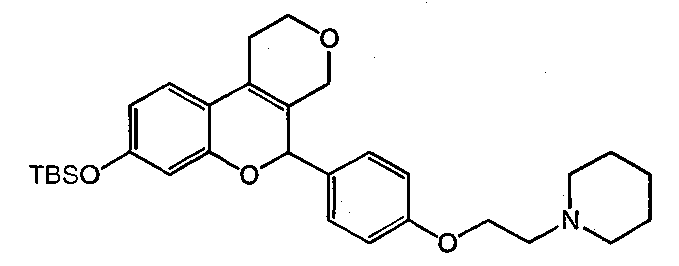

1-[2-(4-iodo-phenoxy)-ethyl]-piperidine (0.894 g, 2.7 mmol) was dissolved in THF (5 mL) and then cooled to -78^{°}C. To the solution was added dropwise n-butyllithium (1.1 mL, 2.5 M in hexane). The solution was stirred at-78^{°}C for 30 min before crude 8-(tert-Butyl-dimethyl-silanyloxy)-1,5-dihydro-2H,4H-pyrano[3,4-c]chromen-5-ol in THF (5 mL) was added. The reaction mixture was then stirred for an additional 30 min after the addition and then quenched with aqueous ammonium chloride and extracted with ethyl acetate. The organic layer was combined, dried over sodium sulfate and concentrated to yield a crude product. The crude product was dissolved into toluene (100 mL) and then cooled to 0^{°}C. TFA (0.28 mL) was then added to the reaction mixture dropwise. The reaction mixture was then stirred at 0^{°}C for 1 hour, diluted with ethyl acetate, washed with 5% NaHCO₃, and then with brine. The organic layer was dried over sodium sulfate and concentrated to yield 1-(2-{4-[8-(tert-Butyl-dimethyl-silanyloxy)-1,5-dihydro-2H,4H-pyrano[3,4-c]chromen-5-yl]-phenoxy}-ethyl)-piperidine as a foam.
LCMS: 6.777 min; m/z, 522 (M + 1)

### Example 12

### 5-[4-(2-Piperidin-1-yl-ethoxy)phenyl]-1,5-dihydro-2H,4H-pyrano[3,4-clchromen-8-ol(Compound ID #1)

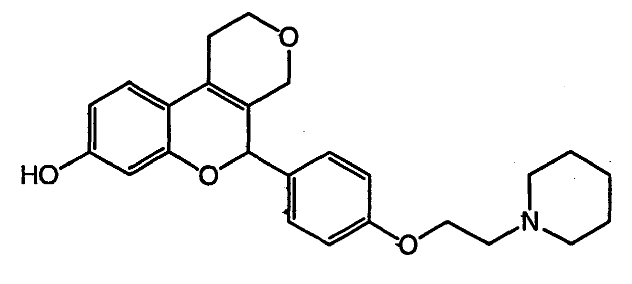

Crude 1-(2-{4-[8-(tert-Butyl-dimethyl-silanyloxy)-1,5-dihydro-2H,4H-pyrano[3,4-c]chromen-5-yl]-phenoxy}-ethyl)-piperidine was dissolved into acetonitrile (5 mL). To the solution was then added pyridine (1 mL) and 70% HF-pyridine (0.5 mL). The reaction mixture was stirred overnight at room temperature, then diluted with ethyl acetate, washed with aqueous 5% NaHCO₃, then with brine. The organic layer was combined, dried over sodium sulfate and concentrated to yield a crude product. The crude product was purified with flash column chromatography eluting with 5:95:1 methanol:dichloromethane:TEA to yield 5-[4-(2-Piperidin-1-yl-ethoxy)-phenyl]-1,5-dihydro-2H,4H-pyrano[3,4-c]chromen-8-ol as a solid.
¹HNMR (CDCl₃, 300 MHz) δ (ppm) 7.2 (d, *J* = 8.4 Hz, 2H), 6.9 (d, *J* = 8.4 Hz, 1 H), 6.7 (d, *J* = 8.4 Hz, 2H), 6.3 (dd, *¹J* = 8.4 Hz, *²J* = 2 Hz, 1 H), 6.2 (d, *J* = 2 Hz, 1 H), 5.45 (s, 1 H), 4.05 (t, *J* = 6 Hz, 2H), 3.95 (t, *J* = 6 Hz, 2H), 3.9 (ABq, *J* = 12 Hz, 2H), 2.8 (m, 2H), 2.6-2.3 (m, 6H), 1.6 (m, 4H), 1.4 (m, 2H);
LCMS: 4.623 min; m/z: 408. (M + 1), 430 (M + 23)

### Preparative Example 13

### 4-(1-Allyl-but-3-enyl)-7-(tert-butyl-dimethyl-silanyloxy)-chromen-2-one and 3-Allyl-4-but-3-enyl-7-(tert-butyl-dimethyl-silanyloxy)-chromen-2-one

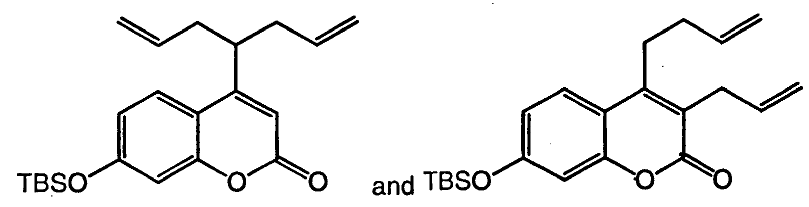

To a solution of 7-(tert-Butyl-dimethyl-silanyloxy)-4-methyl-chromen-2-one (5.8 g, 20 mmol) in THF (200 mL) at -20°C was added slowly 1.0 M LiHMDS in THF (44 mL). The solution was stirred at -20°C for half an hour. The reaction mixture was then transferred through a double-tipped needle into a solution of allyl bromide (3.8 mL, 44 mmol) in THF (60 mL) at -20°C. The reaction mixture was then warmed to room temperature and stirred overnight. The reaction mixture was then quenched with aqueous ammonium chloride and extracted with ethyl acetate. The organic layer was dried over sodium sulfate and concentrated to yield a residue. The residue was purified by flash column chromatography on silica gel eluting with 10% ethyl acetate in hexane to yield two compounds: 4-(1-Allyl-but-3-enyl)-7-(tert-butyl-dimethyl-silanyloxy)-chromen-2-one as a yellow oil, and 3-Allyl-4-but-3-enyl-7-(tert-butyl-dimethyl-silanyloxy)-chromen-2-one as oils.

### 4-(1-Allyl-but-3-enyl)-7-(tert-butyl-dimethyl-silanyloxy)-chromen-2-one

¹HNMR (CDCl₃, 300 MHz) δ (ppm) 7.55 (d, *J* = 8.4 Hz, 1H), 6.75 (m, 2H), 5.9 (m, 2H), 5.1 (m, 4H), 3.35 (d, *J* = 8.4 Hz, 2H), 2.85 (t, *J* = 8.4 Hz, 2H), 2.3 (m, 2H), 1.0 (s, 9H), 0.25 (s, 6H);
¹³CNMR (CDCl₃, 100.6 MHz) δ (ppm) 158.6, 154.5, 151.0, 137.1, 135.1, 125.7, 121.3, 117.5, 116.2, 116.1, 114.1, 108.1, 33.4, 31.8, 28.4, 26.0, 18.6, - 4.0;
LCMS: 5.753 min; m/z, 371 (M + 1), 393 (M + 23), 763 (2M + 23)

### 3-Allyl-4-but-3-enyl-7-(tert-butyl-dimethyl-silanyloxy)-chromen-2-one

¹HNMR (CDCl₃, 300 MHz) δ (ppm) 7.5 (d, *J* = 8.4 Hz, 1 H), 6.8 (m,2H), 6.15 (s, 1 H), 5.7 (m, 2H), 5.05 (m, 4H), 3.2 (m, 1 H), 2.45 (t, *J* = 8.4 Hz, 4H), 1.0 (s, 9H), 0.25 (s, 6H);
¹³CNMR (CDCl₃, 100.6 MHz) δ (ppm) 161.9, 159.5, 158.6, 155.8, 135.1, 125.3, 118.1, 117.5, 13.7, 110.6, 108.5, 39.1, 38.1, 31.9, 25.9, 23.0, 18.6, 14.5, 0.35, -4.0;
LCMS: 5.582 min; m/z, 371 (M + 1), 393 (M + 23), 763 (2M + 23)

### Preparative Example 14

### 3-(tert-Butyl-dimethyl-silanyloxy)-10,11-dihydro-7H-cyclohepta[c]chromen-6-one

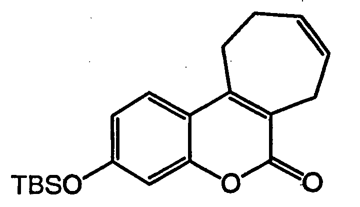

3-Allyl-4-but-3-enyl-7-(tert-butyl-dimethyl-silanyloxy)-chromen-2-one (690 mg, 1.86 mmol) and 20 mg benzylidene-bis(tricyclohexylphosphine)dichlororuthenium (20 mg) were dissolved in DCM (100 mL) and the reaction mixture was stirred at room temperature for 1 hour. Most of the solvent was evaporate and the residue was purified on silica gel column chromatography eluting with 2% ethyl acetate in hexane to yield a colorless oil of 3-(tert-Butyl-dimethyl-silanyloxy)-10,11-dihydro-7H-cyclohepta[c]chromen-6-one.
¹HNMR (CDCl₃, 300 MHz) δ (ppm) 7.55 (d, *J* = 8.4 Hz, 1 H), 6.75 (m, 2H), 5.8-5.55 (m, 2H), 3.5 (m, 2H), 3.15 (t, *J* = 8.4 Hz, 2H), 2.4 (m, 2H), 1.0 (s, 9H), 0.25 (s, 6H);
¹³CNMR (CDCl₃, 100.6 MHz) δ (ppm) 162.0, 158.8, 154.6, 152.3, 129.8, 125.8, 125.0, 124.9, 117.5, 114.1, 108.2, 26.1, 26.0, 24.8, 18.6, -4.0;
LCMS: 5.645 min; m/z, 343 (M + 1), 365 (M + 23), 707 (2M + 23)

### Preparative Example 15

### 3-(tert-Butyl-dimethyl-silanyloxy)-8,9,10,11-tetrahydro-7H-cyclohepta[c]chromen-6-one

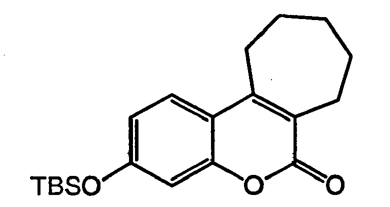

3-(tert-Butyl-dimethyl-silanyloxy)-10,11-dihydro-7H-cyclohepta[c]chromen-6-one (600 mg) and 10% Pd-C (20 mg) were added to methanol (20 mL). The reaction mixture was then shaken overnight under 5 atmospheres of hydrogen. The reaction mixture was then filtrated and concentrated to yield a residue. The residue was purified on silica gel column chromatography eluting with 2% ethyl acetate in hexane to yield a colorless oil of 3-(tert-Butyl-dimethyl-silanyloxy)-8,9,10,11-tetrahydro-7H-cyclohepta[c]chromen-6-one.
¹HNMR (CDCl₃, 300 MHz) δ (ppm) 7.4 (d, *J* = 8.4 Hz, 1 H), 6.7 (m, 2H), 2.9 (m, 4H), 1.85 (m, 2H), 1.7-1.5 (m, 4H), 0.95 (s, 9H), 0.20 (s, 6H);
¹³CNMR (CDCl₃, 100.6 MHz) δ (ppm) 162.7, 158.6, 154.3, 154.1, 125.9, 125.3, 117.3, 114.0, 108.0, 32.4, 28.5, 27.0, 26.2, 26.0, 25.4, 18.6, -4.1;
LCMS: 5.853 min; m/z, 345 (M + 1), 367 (M + 23), 711 (2M + 23)

### Example 16

### 6-[4-(2-Dimethylamino-ethoxy)-phenyl]1-6,7,8,9,10,11-hexahydro-cyclohepta[c]chromen-3-ol (Compound ID #7)

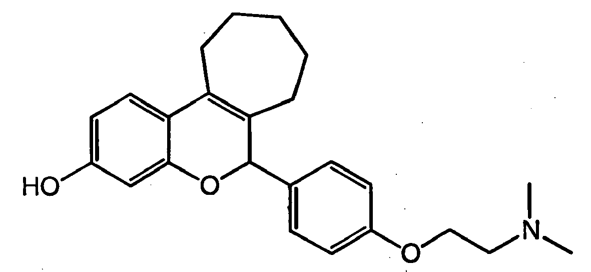

### STEP A: Preparation of 3-(tert-Butyl-dimethyl-silanyloxy)-6,7,8,9,10,11-hexahydro-cyclohepta[c]chromen-6-ol

To a solution of 3-(tert-Butyl-dimethyl-silanyloxy)-8,9,10,11-tetrahydro-7H-cyclohepta[c]chromen-6-one (321 mg, 0.93 mmol) in toluene (9 mL) at - 78°C was added slowly 1.5 M DIBAL-H in toluene (0.62 mL, 0.93 mmol). The reaction was stirred at -78°C for 30 min, then quenched with aqueous ammonium chloride, diluted with ethyl acetate and washed with saturated aqueous sodium potassium tartarate solution. The organic layer was dried over sodium sulfate and concentrated to yield a residue. The residue was azeotropic evaporated with benzene, dried under vacuum and used in the next step without further purification.

### STEP B: Preparation of 6-[4-(2-Dimethylamino-ethoxy)-phenyl]-6,7,8,9,10,11-hexahydro-cyclohepta[c]chromen-3-ol

[2-(4-iodo-phenoxy)-ethyl]-dimethyl-amine (0.924 g, 3.17mmol) was dissolved in THF (5 mL) and cooled to -78°C. To the solution was then added dropwise n-butyllithium (1.12 mL, 2.5 M in hexane). The reaction mixture was stirred at -78°C for 30 min. To the reaction mixture was then added the crude product prepared in Step A above in THF (4 mL). The reaction mixture was then stirred for an additional 30 min, quenched with aqueous ammonium chloride and extracted with ethyl acetate. The organic layer was combined, dried over sodium sulfate and concentrated to yield a residue.

The residue was dissolved into toluene (30 mL) and the mixture cooled to 0°C. To the reaction mixture was then added TFA (0.287 mL) dropwise. The reaction mixture was stirred at 0°C for 1 hour, diluted with ethyl acetate, washed with 5% NaHCO₃, then with brine. The organic layer was dried over sodium sulfate and concentrated to yield a crude product. The crude product was dissolved into acetonitrile (10 mL). The reaction mixture was then added pyridine (1 mL) and 70% HF-pyridine (0.5 mL). The reaction mixture was stirred overnight at room temperature, then diluted with ethyl acetate, washed with aqueous 5% NaHCO₃, then with brine. The organic layer was combined, dried over sodium sulfate and concentrated to yield crude product. The crude product was purified with flash column chromatography eluting with 5:95:1 methanol:dichloromethane:TEA to yield a slight yellow solid of 6-[4-(2-Dimethylamino-ethoxy)-phenyl]-6,7,8,9,10,11-hexahydro-cyclohepta[c]chromen-3-ol.
¹HNMR (CDCl₃, 300 MHz) δ (ppm) 7.2 (d, *J* = 8.4 Hz, 2H), 7.05 (d, *J* = 8.4 Hz, 1H), 6.7 (d, *J* = 8.4 Hz, 2H), 6.3 (dd, *¹J* = 8.4 Hz, *²J* = 2 Hz, 1H), 6.15 (d, *J* = 2 Hz, 1H), 5.44 (s, 1H), 4.0 (t, *J* = 6 Hz, 2H), 2.75 (m, 2H), 2.33 (s, 6H), 2.25-1.9 (m, 4H), 1.8-1.6 (m, 6H).
LCMS: 3.449 min; m/z: 380(M + 1);

### Example 17

### 2-[4-(2-Azepan-1-yl-ethoxy)-phenyl]-4-(2-hydroxy-ethyl)-2H-chromen-7-ol (Compound ID #8)

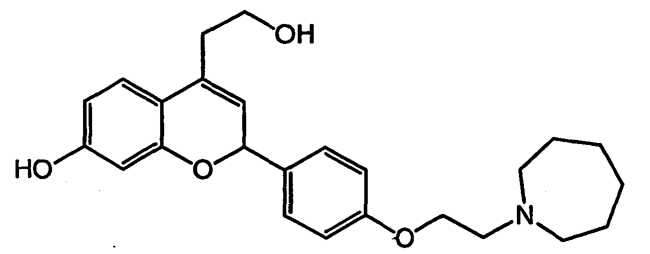

### STEP A: Preparation of 7-(tert-Butyl-dimethyl-silanyloxy)-4-[2-(tert-butyl-dimethyl-silanyloxy)-ethyl]-2H-chromen-2-ol

To a solution of 7-(tert-Butyl-dimethyl-silanyloxy)-4-[2-(tert-butyl-dimethyl-silanyoxy)-ethyl]-chromen-2-one (0.92 g, 2.12 mmol) in toluene (20 mL) at -78°C was added slowly 1.5 M DIBAL-H in toluene (1.7 mL, 2.55 mmol). The reaction mixture was stirred at -78°C for 30 min, then quenched with aqueous ammonium chloride, diluted with ethyl acetate and washed with saturated aqueous sodium potassium tartarate solution. The organic layer was dried over sodium sulfate and concentrated to yield a residue. The residue was azeotropic evaporated with benzene, dried under vacuum to yield a crude producy which was used in the next step without further purification.

### STEP B: Preparation of 2-[4-(2-Azepan-1-yl-ethoxy)-phenyl]-4-(2-hydroxyethyl)-2H-chromen-7-ol

1-[2-(4-iodo-phenoxy)-ethyl]-azepane (2.2 g, 6.37 mmol) was dissolved in THF (15 mL) and then cooled to -78°C. To the solution was then added dropwise n-butyllithium (2.5 mL, 2.5 M in hexane). The solution was stirred at - 78°C for 30 min. To the reaction mixture was then added the crude product prepared in Step A above in THF (5 mL). The reaction mixture was stirred for an additional 30 min, quenched with aqueous ammonium chloride and extracted with ethyl acetate. The organic layer was combined, dried over sodium sulfate and concentrated to yield crude product.
The crude product was dissolved into toluene (60 mL) and then cooled to 0°C. TFA (0.65 mL) was then added dropwise to the reaction mixture. The reaction mixture was stirred at 0°C for 1 hour. The reaction mixture was then diluted with ethyl acetate, washed with 5% NaHCO₃, then with brine. The organic layer was dried over sodium sulfate and concentrated to yield crude product. The crude product was dissolved into acetonitrile (20 mL). To the reaction mixture was then added pyridine (2 mL) and 70% HF-pyridine (1 mL). The reaction mixture was stirred overnight at room temperature, diluted with ethyl acetate, washed with aqueous 5% NaHCO₃, then with brine. The organic layer was combined, dried over sodium sulfate and concentrated to yield crude product. The crude product was purified with flash column chromatography eluting with 5:95:1 methanol:dichloromethane:TEA to yield 2-[4-(2-Azepan-1-yl-ethoxy)-phenyl]-4-(2-hydroxy-ethyl)-2H-chromen-7-ol as a colorless oil.
¹HNMR (CDCl₃, 300 MHz) δ (ppm) 7.3 (d, *J* = 8.4 Hz, 2H), 7.05 (d, *J* = 8.4 Hz, 1H), 6.8 (d, *J* = 8.4 Hz, 2H), 6.35 (dd, *¹J* = 8.4 Hz, *²J* = 2 Hz, 1H), 6.3 (d, *J* = 2 Hz, 1H), 5.75 (d, *J* = 2 Hz, 1H), 5.55 (d, *J* = 2 Hz, 1H), 4.9 (bs, 1H), 4.1 (t, *J* = 6 Hz, 2H), 3.8 (t, *J* = 6 Hz, 1H), 2.95 (t, *J* = 6 Hz, 1H), 2.8 (m, 4H), 2.7 (t, *J* = 6 Hz, 2H), 1.65 (m, 8H).
¹³CNMR (CDCl₃, 100.6 MHz) δ (ppm) 158.9, 155.1, 133.9, 130.9, 128.9, 124.6, 119.8, 114.9, 109.1, 104.5, 65.8, 61.3, 56.4, 56.0, 46.3, 35.0, 27.4, 26.7, 9.9.
LCMS: 4.553 min; m/z: 410 (M + 1);

### Example 18

### 6-[4-(2-Piperidin-1-yl-ethoxy)-phenyl]-6,7,8,9,10,11-hexahydro-cyclohepta[c]chromen-3-ol(Compound ID #9)

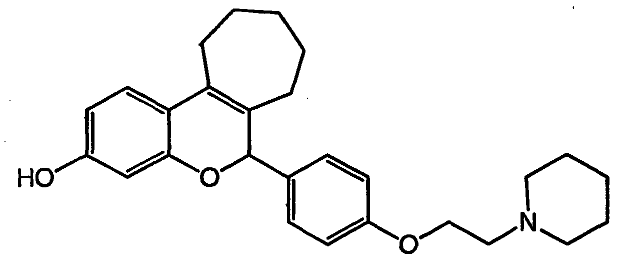

### STEP A: Preparation of 3-(tert-Butyl-dimethyl-silanyloxy)-6,7,8,9,10,11-hexahydro-cyclohepta[c]chromen-6-ol

To a solution of 3-(tert-Butyl-dimethyl-silanyloxy)-8,9,10,11-tetrahydro-7H-cyclohepta[c]chromen-6-one (767 mg, 2.23 mmol) in toluene (20 mL) at - 78°C was added slowly 1.5 M DIBAL-H in toluene (1.78 mL, 2.67 mmol). The reaction mixture was stirred at -78°C for 30 min. then quenched with aqueous ammonium chloride. The reaction mixture was then diluted with ethyl acetate and washed with saturated aqueous sodium potassium tartarate solution. The organic layer was dried over sodium sulfate and concentrated to yield a residue. The residue was azeotropic evaporated with benzene, dried under vacuum to yield a crude product which was used in the next step without further purification.

### STEP B: Preparation of 6-[4-(2-Piperidin-1-yl-ethoxy)-phenyl]-6,7,8,9,10,11--hexahydro-cyclohepta[c]chromen-3-ol

[2-(4-iodo-phenoxy)-ethyl]-piperidine (2.2 g, 6.64mmol) was dissolved in THF (15 mL) and cooled to -78°C. To the solution was added dropwise n-butyllithium (2.7 mL, 2.5 M in hexane). The solution was stirred at -78°C for 30 min. To the reaction mixture was then added the crude product prepared in Step A above in THF (5 mL). The reaction mixture was stirred for an additional 30 min, then quenched with aqueous ammonium chloride and extracted with ethyl acetate. The organic layer was combined, dried over sodium sulfate and concentrated to yield a crude product.
The crude product was dissolved into toluene (60 mL) and then cooled to 0°C. To the reaction mixture was then added dropwise TFA (0.70 mL). The reaction mixture was stirred at 0°C for 1 hour. The reaction mixture was then diluted with ethyl acetate and washed with 5% NaHCO₃, then with brine. The organic layer was dried over sodium sulfate and concentrated to yield a crude product. The crude product was dissolved into acetonitrile (20 mL). To the reaction mixture was then added pyridine (2 mL) and 70% HF-pyridine (1 mL). The reaction mixture was stirred overnight at room temperature. The reaction mixture was diluted with ethyl acetate, washed with aqueous 5% NaHCO₃, then with brine. The organic layer was combined, dried over sodium sulfate and concentrated to yield a crude product. The crude product was purified with flash column chromatography eluting with 5:95:1
methanol:dichloromethane:TEA to yield 6-[4-(2-Piperidin-1-yl-ethoxy)-phenyl]-6,7,8,9,10,11-hexahydro-cyclohepta[c]chromen-3-ol as a yellow foam.
¹HNMR (CDCl₃, 300 MHz) δ (ppm) 7.2 (d, J= 8.4 Hz, 2H), 7.05 (d, *J* = 8.4 Hz, 1H), 6.7 (d, *J* = 8.4 Hz, 2H), 6.3 (dd, *¹J* = 8.4 Hz, *²J* = 2 Hz, 1H), 6.15 (d, *J* = 2 Hz, 1H), 5.44 (s, 1H), 4.0 (t, *J* = 6 Hz, 2H), 2.9-2.3 (m, 6H), 2.3-1.9 (m, 2H), 1.9-1.2 (m, 14H);
¹³CNMR (CDCl₃, 100.6 MHz) δ (ppm) 158.7, 157.0, 152.8, 131.7, 131.2, 130.7, 129.7, 123.1, 116.8, 114.3, 108.2, 104.4, 81.0, 70.5, 65.2, 57.8, 54.9, 45.8, 31.9, 31.6, 27.3, 26.3, 26.1, 25.4, 24.0, 10.7, 7.5;
LCMS: 5.820 min, >97%; m/z: 420 (M + 1), 442 (M + 23).

### Preparative Example 19

### 7-(tert-Butyl-dimethyl-silanyloxy)-4-cyclopent-3-enyl-chromen-2-one

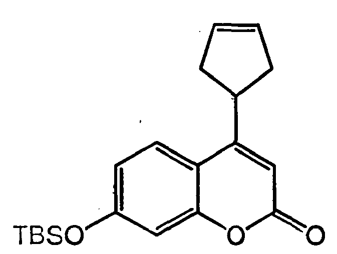

4-(1-Allyl-but-3-enyl)-7-(tert-butyl-dimethyl-silanyloxy)-chromen-2-one (430 mg, 1.16 mmol) and benzylidene-bis(tricyclohexylphosphine)dichlororuthenium (20 mg) were dissolved in DCM (100 mL) and the reaction mixture was stirred at room temperature for 1 hour. Most of the solvent was evaporated and the resulting residue was purified on silica gel column chromatography eluting with 2% ethyl acetate in hexane to yield a colorless oil of 7-(tert-Butyl-dimethyl-silanyloxy)-4-cyclopent-3-enyl-chromen-2-one.
¹HNMR (CDCl₃, 300 MHz) δ (ppm) 7.55 (d, *J* = 8.4 Hz, 1H), 6.75 (m, 2H), 6.15 (s, 1H), 5.8 (s, 2H), 3.75 (m, 1H), 2.9 (dd, ¹*J* = 14.6 Hz, ²*J* = 9.2 Hz, 2H), 2.55 (dd, ¹*J* = 14.4 Hz, ²*J* = 5.2 Hz,2H), 1.0 (s, 9H), 0.25 (s, 6H);
¹³CNMR (CDCl₃, 100.6 MHz) δ (ppm) 162.1, 160.3, 159.3, 155.8, 129.7, 125.7, 117.4, 113.5, 109.5, 108.5, 39.4, 38.6, 25.9, 18.6, -4.0;
LCMS: 5.542 min, > 97%; m/z, 343 (M + 1), 365 (M + 23), 707 (2M + 23)

### Preparative Example 20

### 7-(tert-Butyl-dimethyl-silanyloxy)-4-cyclopentyl-chromen-2-one

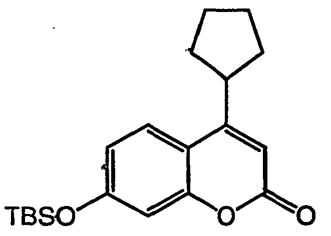

7-(tert-Butyl-dimethyl-silanyloxy)-4-cyclopent-3-enyl-chromen-2-one (388 mg) and 10% Pd-C (20 mg) were added to methanol (10 mL). The reaction mixture was shaken overnight under 5 atmospheres of hydrogen. The reaction mixture was filtrated and concentrated to yield a residue. The residue was purified on silica gel column chromatography eluting with 2% ethyl acetate in hexane to yield a colorless oil of 7-(tert-Butyl-dimethyl-silanyloxy)-4-cyclopentyl-chromen-2-one.
¹HNMR (CDCl₃, 300 MHz) δ (ppm) 7.55 (d, *J* = 8.4 Hz, 1H), 6.7 (m, 2H), 6.2 (s, 1H), 3.3 (m, 4H), 2.1 (m, 2H), 1.9-1.65 (m, 6H), 1.0 (s, 9H), 0.25 (s, 6H);
¹³CNMR (CDCl₃, 100.6 MHz) δ (ppm) 162.3, 160.4, 159.2, 155.6, 128.7, 126.0, 117.3, 114.2, 108.6, 108.3, 41.0, 32.6, 26.0, 25.6, 18.6, -4.0;

### Example 21

### 4-Cyclopentyl-2-[4-(2-piperidin-1-yl-ethoxy)-phenyl]-2H-chromen-7-ol (Compound ID #10)

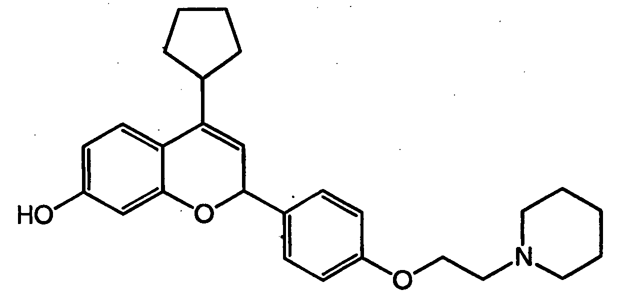

### STEP A: Preparation of 7-(tert-Butyl-dimethyl-silanyloxy)-4-cyclopentyl-2H-chromen-2-ol

To a solution of 7-(tert-Butyl-dimethyl-silanyloxy)-4-cyclopentyl-chromen-2-one (345 mg, 1 mmol) in toluene (10 mL) at -78°C was added slowly 1.5 M DIBAL-H in toluene (0.8 mL, 1.2 mmol). The reaction mixture was stirred at - 78°C for 30 min, then quenched with aqueous ammonium chloride. The reaction mixture was then diluted with ethyl acetate and washed with saturated aqueous sodium potassium tartarate solution. The organic layer was dried over sodium sulfate and concentrate to yield a residue. The residue was azeotropic evaporated with benzene, dried under vacuum to yield a crude product which was used in next step without further purification.

### STEP B: Preparation of 4-Cyclopentyl-2-[4-(2-piperidin-1-yl-ethoxy)-phenyl]-2H-chromen-7-ol

[2-(4-lodo-phenoxy)-ethyl]-piperidine (994 mg, 3 mmol) was dissolved in THF (5 mL) and the reaction mixture was cooled to -78°C. To the reaction mixture was added dropwise n-butyllithium (1.2 mL, 2.5 M in hexane). The reaction mixture was stirred at -78°C for 30 min. To the reaction mixture was then added the crude product prepared in Step A above, in THF (5 mL). The reaction mixture was stirred for an additional 30 min, then quenched with aqueous ammonium chloride and extracted with ethyl acetate. The organic layer was combined, dried over sodium sulfate and concentrated to yield a crude product.
The crude product was dissolved into toluene (30 mL) and the reaction mixture was cooled to 0°C. To the reaction mixture was then added dropwise, TFA (0.3 mL). The reaction mixture was stirred at 0°C for 1 hour, diluted with ethyl acetate and washed with 5% NaHCO₃, then with brine. The organic layer was dried over sodium sulfate and concentrated to yield a crude product. The crude product was dissolved into acetonitrile (10 mL). To the reactiuon mixture was then added pyridine (1 mL) and 70% HF-pyridine (0.5 mL). The reaction mixture was stirred overnight at room temperature, diluted with ethyl acetate and washed with aqueous 5% NaHCO₃, then with brine. The organic layer was combined, dried over sodium sulfate and concentrated to yield a crude product. The crude product was purified with flash column chromatography eluting with 5:95:1 methanol:dichloromethane:TEA to yield a purple solid 4-Cyclopentyl-2-[4-(2-piperidin-1-yl-ethoxy)-phenyl]-2H-chromen-7-ol.
¹HNMR (CDCl₃, 300 MHz) δ (ppm) 7.2 (d, *J* = 8.4 Hz, 2H), 7.15 (d, *J* = 8.4 Hz, 1H), 6.85 (d, *J* = 8.4 Hz, 2H), 6.4 (dd, *¹J* = 8.4 Hz, *²J* = 2 Hz, 1H), 6.3 (d, *J* = 2 Hz, 1H), 5.7 (d, *J* = 2 Hz, 1H), 5.4 (d, *J* = 2 Hz, 1H), 4.1 (t, *J* = 6 Hz, 2H), 3.0 (pentet, *J* = 8.4 Hz, 1H), 2.8 (t, *J* = 6 Hz, 2H), 2.7-2.4 (m, 4H), 2.0 (m, 2H), 1.8-1.4 (m, 12H);
¹³CNMR (CDCl₃, 75.5 MHz) δ (ppm) 158.6, 157.9, 154.9, 137.5, 133.8, 128.6, 124.7, 115.6, 114.9, 114.5, 108.5, 104.0, 77.2, 76.9, 65.4, 57.8, 54.9, 45.8, 40.2, 31.9, 31.6, 25.5, 25., 24., 10.7, ;
LCMS: 5.924 min, >97%;
m/z: 420 (M + 1), 442 (M + 23).

### Example 22

### 3-Hydroxymethyl-4-methyl-2-[4-(2-piperidin-1-yl-ethoxy)-phenyl]-2H-chromen-7-ol (Compound ID #5)

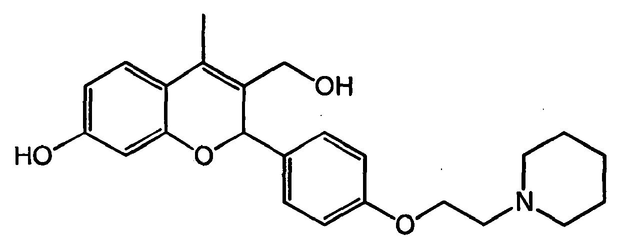

### STEP A: Preparation of 7-(tert-Butyl-dimethyl-silanyloxy)-4-methyl-3-(2-trimethylsilanyl-ethoxymethyl)-2H-chromen-2-ol

To a solution of 7-(tert-Butyl-dimethyl-silanyloxy)-4-methyl-3-(**2-**trimethylsilanyl-ethoxymethyl)-chromen-2-one (1.46 g, 3.36 mmol) in toluene (20 mL) at -78°C was added slowly 1.5 M DIBAL-H in toluene (2.24 mL, 3.36 mmol). The reaction mixture was stirred ay -78°C for 30 min., then quenched with aqueous ammonium chloride. The reaction mixture was then diluted with ethyl acetate (100 mL) and washed with saturated aqueous sodium potassium tartarate solution (4 x 100 ml). The organic layer was dried over sodium sulfate and concentrated to yield a residue. The residue was azeotropic evaporated with benzene, dried under vacuum to yield 7-(tert-Butyl-dimethyl-silanyloxy)-4-methyl-3-(2-trimethylsilanyl-ethoxymethyl)-2H-chromen-2-ol which was used in the next step without further purification.

### STEP B: Preparation of 3-Hydroxymethyl-4-methyl-2-[4-(2-piperidin-1-yl-ethoxy)-phenyl]-2H-chromen-7-ol

1-[2-(4-iodo-phenoxy)-ethyl]-piperidine (3.34 g, 10.08 mmol) was dissolved in THF (30 mL) and the reaction mixture was cooled to -78°C. To the reaction mixture was then added dropwise n-butyllithium (4.0 mL, 2.5 M in hexane). The reaction mixture was stirred at -78°C for 30 min. To the reaction mixture was then added crude 7-(tert-Butyl-dimethyl-silanyloxy)-4-methyl-3-(2-trimethylsilanyl-ethoxymethyl)-2H-chromen-2-ol, prepared in Step A above, in THF (20 mL). The reaction mixture was stirred for an additional 30 min after, then quenched with aqueous ammonium chloride and extracted with ethyl acetate. The organic layer was combined, dried over sodium sulfate and concentrate to yield a crude product. The crude product was dissolved into THF (100 mL). To the reaction mixture was then added triphenylphosphine (969 mg, 3.7 mmol) and DEAD (0.58 mL, 3.7 mmol). The reaction mixture was stirred at ambient temperature for 2 hours. Most of the solvent was evaporated and the resulting residue was dissolved into ethyl acetate and washed with aqueous ammonium chloride, then with brine. The organic layer was dried over sodium sulfate and concentrated to yield a crude product.
The crude product was dissolved into THF (30 mL). To the reaction mixture was then added 1.0 M TBAF in THF (16.8 mL) at room temperature. The reaction mixture was stirred for 3 hours, then and quenched with aqueous NH₄Cl and extracted with 1:1 THF: ethyl acetate. The organic layer was combined, dried over sodium sulfate and concentrated to yield a crude product. The crude product was purified with flash column chromatography eluting with 5:95:1 methanol:dichloromethane:TEA to yield a gray solid of 3-Hydroxymethyl-4-methyl-2-[4-(2-piperidin-1-yl-ethoxy)-phenyl]-2H-chromen-7-ol.
LCMS: 4.227 min, 396 (M + 1);
¹HNMR (CD₃OD, 400 MHz) δ (ppm) 7.2 (d, *J* = 8.4 Hz, 2H), 7.05 (d, *J* = 8.4 Hz, 1H), 6.8 (d, *J* = 8.4 Hz, 2H), 6.3 (dd, *¹J* = 8.4 Hz, *²J* = 2 Hz, 1H), 6.1 (d, *J* = 2 Hz, 1H), 5.8 (s, 1H), 4.45 (d, *J* = 10 Hz, 1H), 4.15 (t, *J* = 6 Hz, 2H), 3.75 (d, *J* = 10 Hz, 1H), 2.95 (t, *J* = 6 Hz, 2H), 2.7 (bs, 4H), 2.1 (s, 3H), 1.65 (m, 4H), 1.45 (m, 2H

### Example 23

### Estrogen Receptor α Flash Plate Assay

This assay monitors binding of radiolabeled estrogen to the estrogen receptor. It is performed on a BioMek 2000 (Beckman). Plates are read in a scintillation counter (Packard TopCount), with decreased counts an indication of binding of a compound to the receptor. The assay was run according to the procedure described by Allan, et al., Anal. Biochem. (1999), 275(2), 243-247.

On day one, 100 µL of Estrogen Screening Buffer (ESB, Panvera) containing 5mM dithiothreitol (DTT, Panvera), 0.5 µg mouse anti-estrogen receptor monoclonal antibody (SRA-1010, Stressgen) and 50 ng purified human estrogen receptor α (Panvera) were added to each well of a 96 well FlashPlate Plus plate crosslinked with goat anti-mouse antibodies (NEN Life Sciences). The plate was sealed and incubated at 4°C overnight.
On day two, each well was washed three times with 200 µL PBS, pH 7.2, at room temperature. To each well was then added 98 µL radiolabeled estrogen (0.5 nM, which equals 6 nCi for a 120 Ci/mmol batch, Amersham), diluted in ESB and 5mM dithiothreitol (DTT). To individual wells were then added 2.5 µL test compound diluted in 30% (v/v) dimethyl sulfoxide/50 mM HEPES, pH 7.5. The wells were mixed three times by aspiration, the plate sealed and incubated at room temperature for one hour. The wells were then counted for 1 min in a TopCount scintillation counter (Packard).

### Example 24

### Estrogen Receptor β Fluorescence Polarization Assay

This assay monitors binding of a fluorescent analog of estrogen (Fluormone ES2, Panvera) to the estrogen receptor. Plates are read in a fluorometer that can be set to polarization mode. A decrease in fluorescence relative to vehicle control is an indication of binding of a compound to the receptor.

It is crucial to avoid introduction of air bubbles into the reaction in each well of the 96 well plate throughout this procedure. (Bubbles on the surface of the reaction disrupt light flow, affecting the polarization reading.) However, it is also crucial to effectively mix the reaction components upon addition to the well.

On ice, a 2X standard mixture of Assay Buffer (Panvera), 10 nM DTT and 40 nM ES2 was prepared. On ice, a 2X reaction mixture of Assay Buffer (Panvera), and 20 nM hER-β (Panvera) and 40 nM ES2 was also prepared.
Dilutions of test compound were prepared in 30% (v/v) dimethyl sulfoxide/50 mM HEPES, pH 7.5. At this point, the dilutions were 40X the final required concentration.

The standard mixture at 50 µL was then added to each well. The reaction mixture at 48 µL was added to all wells. The compound dilution at 2.5 µL was added to the appropriate wells. The reaction mixtures were mixed using a manual pipette, a roll of aluminum foil adhesive cover was placed on the plate and the plate incubated at room temperature for 1 hour.
Each well on the plate was then read in an LjL Analyst with an excitation wavelength of 265 nm and an emission wavelength of 538.

Representative compounds of the present invention were tested according to the procedures described in Examples 23-24, above for binding to the estrogen-α and estrogen-β receptors, with results as listed in Table 2, below.

**Table 2**

| **ID No.** | **ER-α Binding (nM)** | **ER** β**-Binding (nM)** |
|---|---|---|
| **1** | 2200 | >10,000 |
| **3** | 180 | >10,000 |
| **6** | > 10,000 | >10,000 |
| **8** | 500 | 500 |
| **9** | 59 | 690 |
| **10** | 180 | 500 |

### Example 25

As a specific embodiment of an oral composition, 100 mg of the compound prepared as in Example 18 was formulated with sufficient finely divided lactose to provide a total amount of 580 to 590 mg to fill a size O hard gel capsule.

## Claims

1. A compound of formula (I) 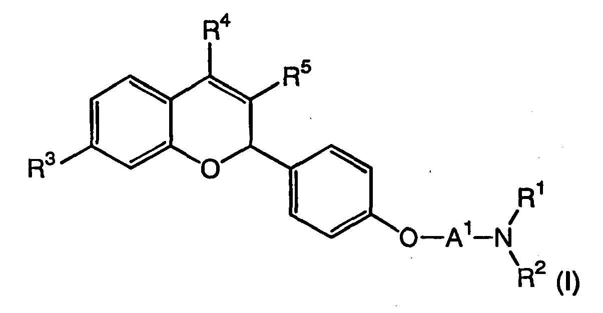 wherein
R¹ and R² are each independently selected from the group consisting of hydrogen and C₁₋₄alkyl;
alternatively, R¹ and R² are taken together with the nitrogen atom to which they are bound to form a 5 to 7 membered ring selected from the group consisting of heteroaryl and heterocycloalkyl; wherein the heteroaryl or heterocycloalkyl group is optionally substituted with one or more substituents independently selected from halogen, hydroxy, C₁₋₄alkyl, C₁₋₄alkoxy, carboxy, amino, C₁₋₄alkylamino, di(C₁₋₄alkyl)amino, nitro or cyano;
A¹ is -C₁₋₄alkyl-;
R³ is selected from the group consisting of hydroxy, C₁₋₄alkoxy, -O-Si(CH₃)₃ and -O-Si(t-butyl)(CH₃)₂;
R⁴ is selected from the group consisting of C₁₋₄alkyl, -C₁₋₄alkyl-OH, -C₁₋₄ alkyl-O-Si(CH₃)₃, -O-Si(t-butyl)(CH₃)₂, C₅₋₇cycloalkyl and C₅₋₇cycloalkenyl;
R⁵ is selected from the group consisting of hydrogen, C₁₋₄alkyl, -C₁₋₄ alkyl-OH, -C₁₋₄alkyl-O-Si(CH₃)₃, -OS-i(t-butyl)(CH₃)₂ and -CH₂-O-CH₂CH₂-Si(CH₃)₃;
alternatively, R⁴ and R⁵ are taken together to form a ring structure selected from 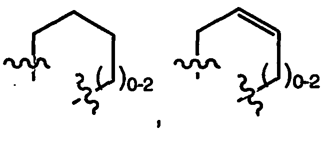 or 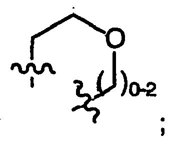 or a pharmaceutically acceptable salt thereof.

2. A compound as in Claim 1, wherein
R¹ is selected from the group consisting of hydrogen and C₁₋₂alkyl;
R² is selected from the group consisting of hydrogen and C₁₋₂alkyl; alternatively, R¹ and R² are taken together with the nitrogen atom to which they are bound to form a 5 to 7 membered heteroaryl or a 5 to 7 membered saturated heterocycloalkyl group;
A¹ is -C₁₋₄alkyl-;
R³ is selected from the group consisting of hydroxy, C₁₋₂alkoxy and -O-Si(t-butyl(CH₃)₂;
R⁴ is selected from the group consisting of -C₁₋₄alkyl, -C₁₋₄alkyl-OH, C₅₋₇ cycloalkyl and C₅₋₇cycloakenyl;
R⁵ is selected from the group consisting of hydrogen, -G₁₋₄alkyl, -C₁₋₄ alkyl-OH and-CH₂-O-CH₂CH₂-Si(CH₃)₃;
alternatively, R⁴ and R⁵ are taken together to form a ring structure selected from 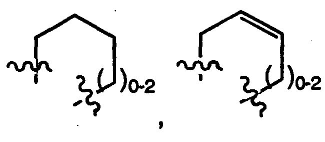 or 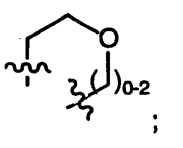 or a pharmaceutically acceptable salt thereof.

3. A compound as in Claim 2, wherein
R¹ is C₁₋₂alkyl;
R² is C₁₋₂alkyl;
alternatively, R¹ and R² are taken together with the nitrogen atom to which they are bound to form a 5 to 7 membered saturated heterocycloalkyl group;
A¹ is -C₁₋₃alkyl-;
R³ is selected from the group consisting of hydroxy and -O-Si(t-butyl(CH₃)₂;
R⁴ is selected from the group consisting of -C₁₋₂alkyl, -C₁₋₂alkyl-OH and C₅₋₆cycloalkyl;
R⁵ is selected from the group consisting of hydrogen, -C₁₋₂alkyl-OH and -CH₂-O-CH₂CH₂-Si(CH₃)₃;
alternatively, R⁴ and R⁵ are taken together to form a ring structure selected from 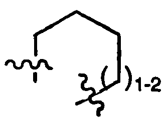 or 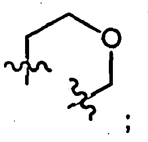 or a pharmaceutically acceptable salt thereof.

4. A compound as in Claim 3, wherein
R¹ is methyl;
R² is methyl;
alternatively, R¹ and R² are taken together with the nitrogen atom to which they are bound to form a group selected from piperidinyl or azepinyl;
A¹ is -CH₂-CH₂-;
R³ is selected from the group consisting of hydroxy and -O-Si(t-butyl(CH₃)₂;
R⁴ is selected from the group consisting of -CH₃, -CH₂CH₂-OH and cyclopentyl;
R⁵ is selected from the group consisting of hydrogen, -CH₂CH₂-OH and -CH₂-O-CH₂-CH₂-Si(CH₃)₃;
alternatively, R⁴ and R⁵ are taken together to form a ring structure selected from 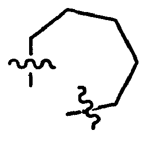 or 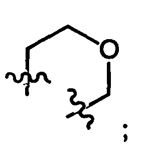 or a pharmaceutically acceptable salt thereof.

5. A compound as in Claim 4, selected from the group consisting of
5-[4-(2-piperidin-1-yl-ethoxy)-phenyl]-1,5-dihydro-2H,4H-pyrano[3,4-c]chromen-8-ol;
4-methyl-2-[4-(2-piperidin-1-yl-ethoxy)-phenyl]-8-(2-trimethylsilanyl-ethoxymethyl)-2H-chromen-7-ol;
4-methyl-(2*S*)-[4-[2-(1-piperidinyl)ethoxy]phenyl]-3[[2-(trimethylsilyl)ethoxy]methyl]- 2*H*-1-benzopyran-7-ol;
4-methyl-2-[4-(2-piperidin-1-yl-ethoxy)-phenyl]-2H-chromen-7-ol;
3-hydroxymethyl-4-methyl-2-[4-(2-piperidin-1-yl-ethoxy)-phenyl]-2H-chromen-7-ol;
4-(2-hydroxy-ethyl)-2-[4-(2-piperidin-1-yl-ethoxy)-phenyl]-2H-chromen-7-ol;
6-[4-(2-dimethylamino-ethoxy)-phenyl]-6,7,8,9,10,11-hexahydro-cyclohepta[c]chromen-3-ol;
2-[4-(2-azepan-1-yl-ethoxy)-phenyl]-4-(2-hydroxy-ethyl)-2H-chromen-7-ol;
6-[4-(2-piperidin-1-yl-ethoxy)-phenyl]-6,7,8,9,10,11-hexahydro-cyclohepta[c]chromen-3-ol;
4-cyclopentyl-2-[4-(2-piperidin-1-yl-ethoxy)-phenyl]-2H-chromen-7-ol;
4-methyl-(2*R*)-[4-[2-(1-piperidinyl)ethoxy]phenyl]-3-[[2-(trimethylsilyl)ethoxy]methyl]- 2*H*-1-benzopyran-7-ol;
1-(2-{4-[8-(tert-Butyl-dimethyl-silanyloxy)-1,5-dihydro-2H,4H-pyrano[3,4-c]chromen-5-yl]-phenoxy}-ethyl)-piperidine;
and pharmaceutically acceptable salts thereof.

6. A compound as in Claim **4**, selected from the group consisting of 4-methyl-(2*S*)-[4-[2-(1-piperidinyl)ethoxy]phenyl]-3-[[2-(trimethylsilyl)ethoxy]methyl]- 2*H*-1-benzopyran-7-ol; and pharmaceutically acceptable salts thereof.

7. A compound as in Claim **4**, selected from the group consisting of 6-[4-(2-Piperidin-1-yl-ethoxy)-phenyl]-6,7,8,9,10,11-hexahydro-cyclohepta[c]chromen-3-ol; and pharmaceutically acceptable salts thereof.

8. A pharmaceutical composition comprising a pharmaceutically acceptable carrier and a compound of any one of claims 1 to 7.

9. A compound of any one of claims 1 to 7 for treating a disorder mediated by at least one estrogen receptor.

10. The compound of claim 9, wherein the estrogen receptor is the α estrogen receptor.

11. The compound of claim 9, wherein the estrogen receptor is the β estrogen receptor.

12. The compound of claim 9, wherein the disorder mediated by an estrogen receptor is selected from the group consisting of hot flushes, vaginal dryness, osteopenia, osteoporosis, hyperlipidemia, loss of cognitive function, degenerative brain diseases, cardiovascular diseases, cerebrovascular diseases, cancer of the breast tissue, hyperplasia of the breast tissue, cancer of the endometrium, hyperplasia of the endometrium, cancer of the cervix, hyperplasia of the cervix, cancer of the prostate, hyperplasia of the prostate, endometriosis, uterine fibroids, osteoarthritis and contraception.

13. The compound of claim 9, wherein the disorder mediated by at least one estrogen receptor is selected from the group consisting of osteoporosis, hot flushes, vaginal dryness, breast cancer and endometriosis.

14. A pharmaceutical composition of claim 8 for treating a disorder mediated by at least one estrogen receptor.

15. A compound of any one of claims 1 to 7 for use in co-therapy with a progestogen or a progestogen antagonist, for contraception.

16. The use of a compound as claimed in any one of claims 1 to 7 for the preparation of a medicament for treating: (a) hot flushes, (b) vaginal dryness, (c) osteopenia, (d) osteoporosis, (e) hyperlipidemia, (f) loss of cognitive function, (g) degenerative brain diseases, (h) cardiovascular diseases, (i) cerebrovascular diseases, (j) cancer of the breast tissue, (k) hyperplasia of the breast tissue, (l) cancer of the endometrium, (m) hyperplasia of the endometrium, (n) cancer of the cervix, (o) hyperplasia of the cervix, (p) cancer of the prostate, (q) hyperplasia of the prostate, (r) endometriosis, (s) uterine fibroids, (t) osteoarthritis, and (u) for contraception in a subject in need thereof.

17. The use of a compound as claimed in any one of claims 1 to 7 for the manufacture of a medicament for treating a disorder mediated by at least one estrogen receptor.

## Patentansprüche

1. Verbindung der Formel (I) 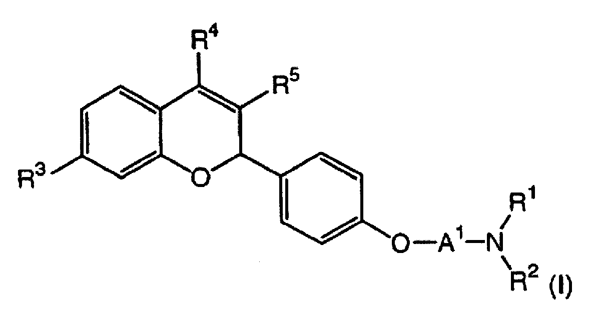 wobei
R¹ und R² jeweils unabhängig ausgewählt sind aus der Gruppe bestehend aus Wasserstoff und C₁₋₄Alkyl;
alternativ dazu R¹ und R² mit dem Stickstoffatom zusammen genommen sind, an welches sie gebunden sind, um einen 5- bis 7-Ring zu bilden, der ausgewählt ist aus der Gruppe bestehend aus Heteroaryl und Heterocycloalkyl; wobei die Heteroaryl- oder die Heterocycloalkyl-Gruppe optional substituiert ist mit einem oder mehreren Substituenten, die unabhängig ausgewählt sind aus Halogen, Hydroxy, C₁₋₄Alkyl, C₁₋₄Alkoxyl, Carboxy, Amino, C₁₋₄ Alkylamino, Di(C₁₋₄Alkyl)Amino, Nitro oder Cyano.
A¹-C₁₋₄Alkyl- ist;
R³ ausgewählt ist aus der Gruppe bestehend aus Hydroxy, C₁₋₄Alkoxy, -O-Si(CH₃)₃ und -O-Si(t-Butyl)(CH₃)₂;
R⁴ ausgewählt ist aus der Gruppe bestehend aus C₁₋₄Alkyl, -C₁₋₄Alkyl-OH, -C₁₋₄Alkyl-O-Si(CH₃)₃, -O-Si(t-Butyl)(CH₃)₂, C₅₋₇Cycloalkyl und C₅₋₇Cycloalkenyl;
R⁵ ausgewählt ist aus der Gruppe bestehend aus Wasserstoff, C₁₋₄Akyl, -C₁₋₄Alkyl-OH, -C₁₋₄Alkyl-O-Si(CH₃)₃, -O-Si(t-Butyl)(CH₃)₂ und-CH₂-O-CH₂CH₂-Si(CH₃)₃;
alternativ dazu R⁴ und R⁵ zusammen genommen sind, um eine Ringstruktur zu bilden, ausgewählt aus 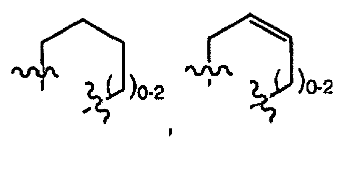 oder 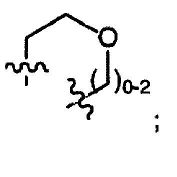 oder ein pharmazeutisch akzeptables Salz davon.

2. Verbindung nach Anspruch 1, wobei
R¹ ausgewählt ist aus der Gruppe bestehend aus Wasserstoff und C₁₋₂Alkyl;
R² ausgewählt ist aus der Gruppe bestehend aus Wasserstoff und C₁₋₂Alkyl; alternativ dazu R¹ und R² mit dem Stickstoffatom zusammen genommen sind, an welches sie gebunden sind, um eine 5- bis 7-Heteroarylgruppe oder eine gesättigte 5- bis 7-Heterocycloakylgruppe zu bilden;
A¹-C₁₋₄Alkyl- ist;
R³ ausgewählt ist aus der Gruppe bestehend aus Wasserstoff, C₁₋₂Alkoxy und -O-Si(t-Butyl)(CH₃)₂;
R⁴ ausgewählt ist aus der Gruppe bestehend aus -C₁₋₄Alkyl, -C₁₋₄Alkyl-OH, C₅₋₇ Cycloalkyl und C₅₋₇Cycloalkenyl;
R⁵ ausgewählt ist aus der Gruppe bestehend aus Wasserstoff, -C₁₋₄Alkyl, -C₁₋₄Alkyl-OH und -CH₂O-CH₂CH₂-Si(CH₃)₃;
alternativ dazu R⁴ und R⁵ zusammen genommen sind, um eine Ringstruktur zu bilden, ausgewählt aus 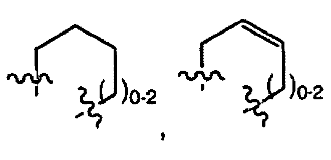 oder 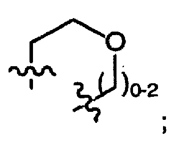
oder ein pharmazeutisch akzeptables Salz davon.

3. Verbindung nach Anspruch 2, wobei
R¹ C₁₋₂Alkyl ist;
R² C₁₋₂Alkyl ist;
alternativ dazu R¹ und R² mit dem Stickstoffatom zusammen genommen sind, an welches sie gebunden sind, um eine gesättigte 5- bis 7-Heterocycloalkyl-Gruppe zu bilden.
A¹ -C₁₋₃Alkyl ist;
R³ ausgewählt ist aus der Gruppe bestehend aus Hydroxy und O-Si(t-Butyl)(CH₃)₂;
R⁴ ausgewählt ist aus der Gruppe bestehend aus -C₁₋₂Alkyl, -C₁₋₂Alkyl-OH und C₅₋₆ Cycloalkyl;
R⁵ ausgewählt ist aus der Gruppe bestehend aus Wasserstoff, -C₁₋₂Alkyl-OH und - CH₂-O-CH₂CH₂-Si(CH₃)₃;
alternativ dazu R⁴ und R⁵ zusammen genommen sind, um eine Ringstruktur zu bilden, ausgewählt aus 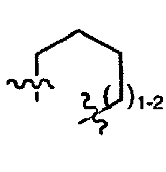 oder 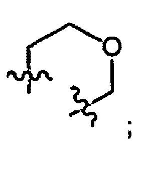
oder ein pharmazeutisch akzeptables Salz davon.

4. Verbindung nach Anspruch 3, wobei
R¹ Methyl ist;
R² Methyl ist;
alternativ dazu R¹und R² mit dem Stickstoffatom zusammen genommen sind, an welches sie gebunden sind, um eine Gruppe zu bilden, ausgewählt aus Piperidinyl oder Azepinyl;
A¹ - CH₂-CH₂- ist;
R³ ausgewählt ist aus der Gruppe bestehend aus Hydroxy und -O-Si(t-Buthyl)(CH₃)₂;
R⁴ ausgewählt ist aus der Gruppe bestehend aus -CH₃, -CH₂CH₂-OH und Cyclopentyl;
R⁵ ausgewählt ist aus der Gruppe bestehend aus Wasserstoff, -CH₂CH₂-OH und -CH₂-O-CH₂-CH₂-Si(CH₃)₃;
alternativ dazu R⁴ und R⁵ zusammen genommen sind, um eine Ringstruktur zu bilden, ausgewählt aus 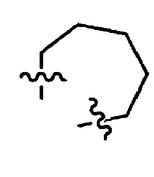 oder 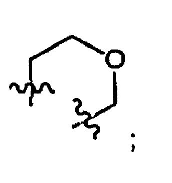
oder ein pharmazeutisch akzeptables Salz davon.

5. Verbindung nach Anspruch 4, ausgewählt aus der Gruppe bestehend aus
- 5-[4-(2-Piperidin-1-yl-ethoxy)-phenyl]-1,5-dihydro-2H, 4H-pyrano[3,4-c]chromen-8-ol;
- 4-Methyl-2-[4-(2-Piperidin-1-yl-ethoxy)-phenyl]-3-(2-trimethylsilanyl-ethoxymethyl)-2H-chromen-7-ol;
- 4-Methyl-(2*S*)-[4-[2-(1-Piperidinyl)ethoxy]-phenyl]-3-[[2-(trimethylsilyl)ethoxy]methyl]-2*H*-1-benzopyran-7-ol;
- 4-Methyl-2-[4-(2-Piperidin-1-yl-ethoxy)-phenyl]-2H-chromen-7-ol;
- 3-Hydroxymethyl-4-methyl-2-[4-(2-piperidin-1-yl-ethoxy)-phenyl]-2H-chromen-7-ol;
- 4-(2-Hydroxy-Ethyl)-2-[4-(2-piperidin-1-yl-ethoxy)-phenyl]-2H-chromen-7-ol;
- 6-[4-(2-Dimethylamino-ethoxy)-phenyl]-6,7,8,9,10,11-hexahydrocyclohepta[c]chromen-3-ol;
- 2-[4-(2-Azepan-1-yl-ethoxy)-phenyl]-4-(2-hydroxy-ethyl)-2H-chromen-7-ol;
- 6-[4-(2-Piperidin-1-yl-ethoxy)-phenyl]-6,7,8,9,10,11-hexahydrocyclohepta[c]chromen-3-ol;
- 4-Cyclophentyl-2-[4-(2-Piperidin-1-yl-ethoxy)-phenyl]-2H-chromen-7-ol;
- 4-Methyl-(2R)-[4-[2-(1-Piperidinyl)ethoxy]phenyl]-3-[[2-(trimethylsilyl)ethoxy]methyl]-2*H*-1-benzopyran-7-ol;
- 1-(2-{4-[8-tert-Butyl-dimethyl-silanyloxy)-1,5-dihydro-2H,4H-pyrano[3,4-c]chromen-5-yl]-phenoxy}-ethyl)-piperidin;
und pharmazeutisch akzeptablen Salzen davon.

6. Verbindung nach Anspruch 4, ausgewählt aus der Gruppe bestehend aus 4-Methyl-(2*S*)-[4-[2-(1-Piperidinyl)ethoxy]phenyl]-3-[[2-trimethylsilyl)ethoxy]methyl]-2*H*-1-benzopyran-7-ol, und pharmazeutisch akzeptablen Salzen davon.

7. Verbindung nach Anspruch 4, ausgewählt aus der Gruppe bestehend aus 6-[4-(2-Piperidin-1-yl-ethoxy)-phenyl]-6,7,8,9,10,11-hexahydro-cyclohetpa[c]chromen-3-ol; und pharmazeutisch akzeptablen Salzen davon.

8. Pharmazeutische Zusammensetzung, umfassend einen pharmazeutisch akzeptablen Träger und eine Verbindung nach einem der Ansprüche 1 bis 7.

9. Verbindung nach einem der Ansprüche 1 bis 7 für die Behandlung einer Erkrankung, die von mindestens einem Östrogen-Rezeptor vermittelt wird.

10. Verbindung nach Anspruch 9, wobei der Östrogen-Rezeptor der α-Östrogen-Rezeptor ist.

11. Verbindung nach Anspruch 9, wobei der Östrogen-Rezeptor der β-Östrogen-Rezeptor ist.

12. Verbindung nach Anspruch 9, wobei die Erkrankung, die durch einen Östrogen-Rezeptor vermittelt wird, ausgewählt ist aus der Gruppe bestehend aus Hitzewallungen, vaginaler Trockenheit, Osteopenie, Osteroporosis, Hyperlipidämie, Verlust an kognitiver Funktion, degenerativen Hirnerkrankungen, kardiovaskulären Erkrankungen, zerebrovaskulären Erkrankungen, Krebs des Brustgewebes, Hyperplasie des Brustgewebes, Krebs des Endometriums, Hyperplasie des Endometriums, Krebs des Zervix, Hyperplasie des Zervis, Krebs der Prostata, Hyperplasie der Prostata, Endometriose, Uterus-Fibrom, Osteoarthritis und Kontrazeption.

13. Verbindung nach Anspruch 9, wobei die Erkrankung, die durch mindestens einen Östrogen-Rezeptor vermittelt wird, ausgewählt ist aus der Gruppe bestehend aus Osteroporosis, Hitzewallungen, vaginaler Trockenheit, Brustkrebs und Endometriose.

14. Pharmazeutische Zusammensetzung nach Anspruch 8 für die Behandlung einer Erkrankung, die durch mindestens einen Östrogen-Rezeptor vermittelt wird.

15. Verbindung nach einem der Ansprüche 1 bis 7 für die Verwendung in einer Co-Therapie mit einem Progestogen oder einem Progestogen-Antagonist für die Kontrazeption.

16. Verwendung einer Verbindung, wie in einem der Ansprüche 1 bis 7 beansprucht, für die Herstellung eines Medikamentes für die Behandlung von: (a) Hitzewallungen, (b) vaginaler Trockenheit, (c) Osteopenie, (d) Osteoporosis, (e) Hyperlipidämie, (f) Verlust an kognitiver Funktion, (g) degenerativen Hirnerkrankungen, (h) kardiovaskulären Erkrankungen, (i) zerebrovaskulären Erkrankungen, (j) Krebs des Brustgewebes, (k) Hyperplasie des Brustgewebes, (1) Krebs des Endometriums, (m) Hyperplasie des Endometriums, (n) Krebs des Zervix, (o) Hyperplasmie des Zervix, (p) Krebs der Prostata, (q) Hyperplasie der Prostata, (r) Endometriose, (s) Uterus-Fibrom, (t) Osteoarthritis und (u) für die Kontrazeption in einem Subjekt, welches Bedarf dafür hat.

17. Verwendung einer Verbindung, wie in einem der Ansprüche 1 bis 7 beansprucht, für die Herstellung eines Medikaments für die Behandlung einer Erkrankung, die durch mindestens einen Östrogen-Rezeptor vermittelt wird.

## Revendications

1. Composé de formule (I) 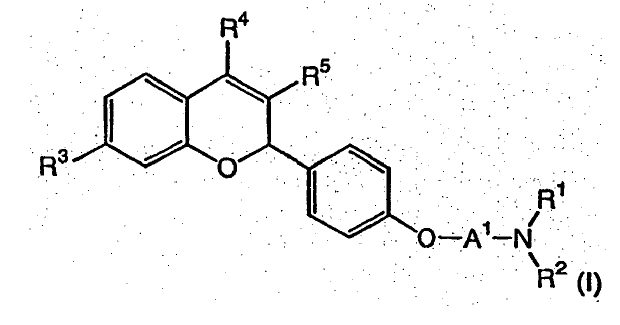 où
R¹ et R² sont chacun indépendamment sélectionnés dans le groupe consistant en hydrogène et alkyleC₁₋₄;
alternativement, R¹ et R² sont pris ensemble avec l'atome d'azote auquel ils sont liés pour former un cycle à 5 à 7 membres sélectionné dans le groupe consistant en hétéroaryle et hétérocycloalkyle; où le groupe hétéroaryle ou hétérocycloalkyle est facultativement substitué par un ou plusieurs substituants indépendamment sélectionnés parmi halogène, hydroxy, alkyleC₁₋₄, alcoxyC₁₋₄, carboxy, amino, alkylaminoC₁₋₄, dialkylaminoC₁₋₄, nitro ou cyano;
A¹ est alkyleC₁₋₄;
R³ est sélectionné dans le groupe consistant en hydroxy, alcoxyC₁₋₄, -O-Si(CH₃)₃ et -O-Si(t-butyl) (CH₃)₂;
R⁴ est sélectionné dans le groupe consistant en alkyleC₁₋₄, -alkylC₁₋₄-OH, alkylC₁₋₄-O-Si(CH₃)₃, -O-Si(t-butyl) (CH₃)₂, cycloalkyleC₅₋₇ et cycloalcényleC₅₋₇;
R⁵ est sélectionné dans le groupe consistant en hydrogène, alkyleC₁₋₄-, alkylC₁₋₄-OH, -alkylC₁₋₄-O-Si(CH₃)₃, - O-Si (t-butyl) (CH₃)₂, et -CH₂-O-CH₂CH₂-Si (CH₃)₃;
Alternativement, R⁴ et R⁵ sont pris ensemble pour former une structure cylique sélectionnée parmi 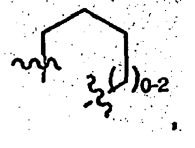 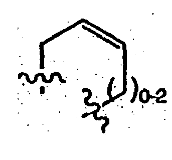 ou 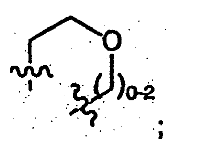
ou un sel pharmaceutiquement acceptable.

2. Composé tel qu'à la revendication 1, où
R¹ est sélectionné dans le groupe consistant en hydrogène et alkyleC₁₋₂;
R² est sélectionné dans le groupe consistant en hydrogène et alkyleC₁₋₂;
alternativement, R¹ et R² sont pris ensemble avec l'atome d'azote auquel ils sont liés pour former un hétéroaryle à 5 à 7 membres ou un groupe hétérocycloalkyle saturé à 5 à 7 membres;
A¹ est -alkyleC₁₋₄₋;
R³ est sélectionné dans le groupe consistant en hydroxy, alcoxyC₁₋₂ et -O-Si(t-butyl(CH₃)₂;
R⁴ est sélectionné dans le groupe consistant en alkyleC₁₋₄, -alkylC₁₋₄-OH, cycloalkyleC₅₋₇ et cycloalcényle C₅₋₇ ;
R⁵ est sélectionné dans le groupe consistant en hydrogène, -alkyleC₁₋₄, -alkylC₁₋₄-OH et -CH₂-O-CH₂CH₂-Si(CH₃)₃;
alternativement, R⁴ et R⁵ sont pris ensemble pour former une structure cyclique sélectionnée parmi 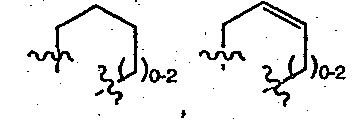 ou 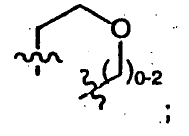
ou un sel pharmaceutiquement acceptable.

3. Composé selon la revendication 2, où
R¹ est alkyleC₁₋₂;
R² est alkyleC₁₋₂;
alternativement, R¹ et R² sont pris ensemble avec l'atome d'azote auquel ils sont liés pour former un groupe hétérocycloalkyle saturé de 5 à 7 membres;
A¹ est -alkyleC₁₋₃;
R³ est sélectionné dans le groupe consistant en hydroxy, alcoxyC₁₋₄ et -O-Si(t-butyl) (CH₃)₂;
R⁴ est sélectionné dans le groupe consistant en -alkyleC₁₋₄; -alkylC₁₋₄-OH, et cycloalkyleC₅₋₇;
R⁵ est sélectionné dans le groupe consistant en hydrogène, -alkylC₁₋₄-OH, et -CH₂-O-CH₂CH₂-Si(CH₃)₃;
alternativement, R⁴ et R⁵ sont pris ensemble pour former une structure cyclique sélectionnée parmi 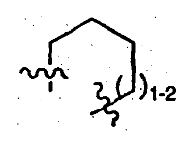 ou 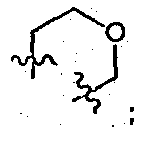
ou un sel pharmaceutiquement acceptable.

4. Composé selon la revendication 3, où
R¹ est méthyle;
R² est méthyle;
alternativement, R¹ et R² sont pris ensemble avec l'atome d'azote auquel ils sont liés pour former un groupe sélectionné parmi pipéridinyle ou azépinyle;
A¹ est -CH₂-CH₂-;
R³ est sélectionné dans le groupe consistant en hydroxy et -O-Si(t-butyl(CH₃)₂;
R⁴ est sélectionné dans le groupe consistant en -CH₃, -CH₂CH₂-OH et cyclopentyle;
R⁵ est sélectionné dans le groupe consistant en hydrogène, -CH₂CH₂-OH et -CH₂-O-CH₂-CH₂-Si(CH₃)₃;
alternativement, R⁴ et R⁵ sont pris ensemble pour former une structure cyclique sélectionnée parmi 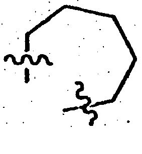 ou 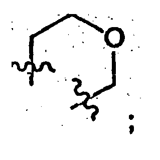
ou un sel pharmaceutiquement acceptable.

5. Composé selon la revendication 4, sélectionné dans le groupe consistant en
5-[4-(2-pipéridin-1-yl-éthoxy)-phényl]-1,5-dihydro-2H,4H-pyrano[3,4-c]chromén-8-ol;
4-méthyl-2-[4-(2-pipéridin-1-yl-éthoxy)-phényl]-3-(2-triméthylsilyl-éthoxyméthyl)-2H-chromén-7-ol;
4-méthyl-(2*S*)-[4-[2-(1-pipéridinyl)éthoxy]-phényl-3-[[2-(triméthylsilyl)éthoxy]méthyl]-2*H*-1-benzopyran-7-ol;
4-méthyl-2-[4-(2-pipéridin-1-yl-éthoxy)-phényl]-2H-chromén-7-ol;
3-hydroxyméthyl-4-méthyl-2-[4-(2-pipéridin-1-yl-éthoxy)-phényl]-2H-chromén-7-ol;
4-(2-hydroxy-éthyl)-2-[4-(2-pipéridin-1-yl-éthoxy)-phényl]-2H-chromén-7-ol;
6-[4-(2-diméthylamino-éthoxy)-phényl]-6,7,8,9,10,11-hexahydro-cyclohepta[c]chromén-3-ol;
2-[4-(2-azépan-1-yl-éthoxy)-phényl]-4-(2-hydroxy-éthyl)-2H-chromén-7-ol;
6-[4-(2-pipéridin-1-yl-éthoxy)-phényl]-6,7,8,9,10,11-hexahydro-cyclohepta[c]chromén-3-ol;
4-cyclopentyl-2-[4-(2-pipéridin-1-yl-éthoxy)-phényl]-2H-chromén-7-ol;
4-méthyl-(2*H*)-[4-[2-(1-pipéridinyl)éthoxy]phenyl-3-[[2-(triméthylsilyl)éthoxy]methyl]-2*H*-1-benzopyran-7-ol;
1-(2-[4-[8-(tert-Butyl-diméthyl-silanyloxy)-1,5-dihydro-2H,4H-pyrano[3,4-c]chromén-5-yl]-phénoxy)-éthyl)-pipéridine;
et leurs sels pharmaceutiquement acceptables.

6. Composé selon la revendication 4, sélectionné dans le groupe consistant en 4-méthyl-(2*S*)-[4-[2-(1-pipéridinyl)éthoxy]phényl]-3-[[2-(triméthylsilyl)éthoxy] méthyl]-2*H*-1-benzopyran-7-ol; et ses sels pharmaceutiquement acceptables.

7. Composé selon la revendication 4 sélectionné dans le groupe consistant en 6-[4-(2-pipéridin-1-yl-éthoxy)-phényl]-6,7,8,9,10,11-hexahydro-cyclohepta[c]chromén-3-ol; et ses sels pharmaceutiquement acceptables.

8. Composition pharmaceutique comprenant un support pharmaceutiquement acceptable et un composé de l'une quelconque des revendications 1 à 7.

9. Composé de l'une quelconque des revendications 1 à 7 pour le traitement d'un trouble provoqué par au moins un récepteur d'oestrogène.

10. Composé de la revendication 9, où le récepteur d'oestrogène est le récepteur d'oestrogène α.

11. Composé de la revendication 9, où le récepteur d'oestrogène est le récepteur d'oestrogène β.

12. Composé de la revendication 9, où le trouble provoqué par un récepteur d'oestrogène est sélectionné dans le groupe consistant en bouffées de chaleur, sécheresse vaginale, ostéopénie, ostéoporose, hyperlipidémie, perte de fonction cognitive, maladies dégénératives du cerveau, maladies cardiovasculaires, maladies cérébrovasculaires, cancer du tissu du sein, hyperplasie du tissu du sein, cancer de l'endomètre, hyperplasie de l'endomètre, cancer du col de l'utérus, hyperplasie du col de l'utérus, cancer de la prostate, hyperplasie de la prostate, endométriose, fibroïdes utérins, ostéoarthrite et contraception.

13. Composé de la revendication 9, où le trouble provoqué par au moins un récepteur d'oestrogène est sélectionné dans le groupe consistant en ostéoporose, bouffées de chaleur, sécheresse vaginale, cancer du sein et endométriose.

14. Composition pharmaceutique de la revendication 8 pour le traitement d'un trouble provoqué par au moins un récepteur d'oestrogène.

15. Composé de l'une quelconque des revendications 1 à 7 à utiliser en co-thérapie avec un progestogène ou un antagoniste de progestogène pour la contraception.

16. Utilisation d'un composé selon l'une quelconque des revendications 1 à 7 pour la préparation d'un médicament pour le traitement de: (a) les bouffées de chaleur, (b) la sécheresse vaginale, (c) l'ostéopénie, (d) l'ostéoporose, (e) l'hyperlipidémie, (f) la perte de fonction cognitive, (g) les maladies dégénératives du cerveau, (h) les maladies cardiovasculaires, (i) les maladies cérébrovasculaires, (j) le cancer du tissu du sein, (k) hyperplasie du tissu du sein, (1) le cancer de l'endomètre, (m) l'hyperplasie de l'endomètre, (n) le cancer du col de l'utérus, (o) l'hyperplasie du col de l'utérus, (p) le cancer de la prostate, (q) l'hyperplasie de la prostate, (r) l'endométriose, (s) les fibroïdes utérins, (t) l'ostéoarthrite, et (u) pour la contraception chez un sujet le nécessitant.

17. Utilisation d'un composé selon l'une quelconque des revendications 1 à 7 à utiliser dans la fabrication d'un médicament pour le traitement d'un trouble provoqué par au moins un récepteur d'oestrogène.
